(19) Europäisches Patentamt
European Patent Office
Office européen des brevets

(11) EP 2 525 771 B1

(12) **EUROPÄISCHE PATENTSCHRIFT**

(45) Veröffentlichungstag und Bekanntmachung des Hinweises auf die Patenterteilung:
**13.07.2016 Patentblatt 2016/28**

(21) Anmeldenummer: **10794992.7**

(22) Anmeldetag: **17.12.2010**

(51) Int Cl.:
***A61K 8/00*** *(2006.01)* ***A61Q 5/00*** *(2006.01)*
***C08G 77/04*** *(2006.01)* ***C08G 77/26*** *(2006.01)*
***C08G 77/388*** *(2006.01)*

(86) Internationale Anmeldenummer:
**PCT/EP2010/070071**

(87) Internationale Veröffentlichungsnummer:
**WO 2011/088937 (28.07.2011 Gazette 2011/30)**

# (54) NEUARTIGE POLYSILOXANE MIT QUATÄREN AMMONIUMGRUPPEN, VERFAHREN ZU DEREN HERSTELLUNG UND DEREN VERWENDUNG IN REINIGENDEN UND PFLEGENDEN FORMULIERUNGEN

NOVEL POLYSILOXANES HAVING QUATERNARY AMMONIUM GROUPS, METHOD FOR PRODUCING SAME AND USE THEREOF IN FORMULATIONS FOR CLEANSING AND CARE

NOUVEAUX POLYSILOXANES À GROUPES AMMONIUMS QUATERNAIRES, LEUR PROCÉDÉ DE FABRICATION ET LEUR UTILISATION POUR DES FORMULATIONS PURIFIANTES ET DE SOINS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.01.2010 DE 102010000993**

(43) Veröffentlichungstag der Anmeldung:
**28.11.2012 Patentblatt 2012/48**

(73) Patentinhaber: **Evonik Degussa GmbH**
**45128 Essen (DE)**

(72) Erfinder:
- **HERRWERTH, Sascha**
  **45134 Essen (DE)**
- **HARTUNG, Christian**
  **45133 Essen (DE)**
- **WINTER, Patrick**
  **45473 Mülheim an der Ruhr (DE)**
- **FERENZ, Michael**
  **45147 Essen (DE)**
- **HENNING, Frauke**
  **45259 Essen (DE)**

(56) Entgegenhaltungen:
**EP-A1- 1 561 770** **US-A- 5 152 984**
**US-A1- 2005 255 073** **US-A1- 2008 305 065**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 525 771 B1

Printed by Jouve, 75001 PARIS (FR)

## Beschreibung

Gebiet der Erfindung:

Die Erfindung betrifft neuartige Polysiloxane mit quatären Ammoniumgruppen sowie ein Verfahren zu deren Herstellung. Sie betrifft ferner die Verwendung dieser Polymeren als Pflegewirkstoff in Formulierungen für die Pflege und Reinigung von Haut und Hautanhangsgebilden, wie beispielsweise als Konditioniermittel für Haare, sowie in Reinigern und Pflegemitteln in Haushalt und Industrie.

Stand der Technik:

Polysiloxane mit quatären Ammoniumgruppen und deren Anwendung als Additive für Haarpflege oder Textilweichmacher sind aus der Patentliteratur bekannt.

So werden zum Beispiel in DE 14 93 384, EP 0017122 und US 4895964 Strukturen beschrieben, bei denen Siloxane mittelständig mit statistisch über das Polymer verteilten quatären Ammoniumgruppen modifiziert sind. Diese Verbindungen haben den Nachteil, dass sie keinen ausgeprägten Siliconcharakter besitzen und eine gute Wirksamkeit als Konditioniermittel für z.B. Haare oder Textilien nicht zu beobachten ist.

Einen ausgeprägteren Siliconcharakter haben kationische Polysiloxane, wie sie in DE 37 19 086 und EP 0 294 642 beschrieben werden. Bei den in der DE 37 19 086 und die in der EP 0 294 642 beschreiben Strukturen sind die quatären Funktionen endständig an das Polysiloxan gebunden. Derartige Verbindungen bieten Vorteile hinsichtlich ihrer Wirkung als Konditioniermittel sowohl für Haare und Textilien als auch für harte Oberflächen. Die Verwendung solcher Verbindungen in kosmetischen Formulierungen ist z.B. in EP0530974, EP617607, EP1080714, WO2001082879 und US6207141 beschrieben worden.

Allerdings besitzen die dort beschriebenen Strukturen nur zwei kationische Gruppen. Aufgrund der verhältnismäßig geringen Substantivität ist die Affinität der Polysiloxane zu bestimmten Oberflächen verhältnismäßig gering.

Aus der DE-OS 33 40 708 sind polyquatäre Polysiloxan-Polymere bekannt. Polyquatäre Polysiloxan-Polymere dieses Typs weisen die oben beschriebenen Nachteile nicht auf. Der praktischen Verwendung dieser Verbindungen steht jedoch deren aufwändiges Herstellverfahren entgegen. Die Verbindungen sind in wirtschaftlich nicht vertretbaren Ausbeuten von <60% der Theorie herstellbar.

Menschliches Haar ist täglich den verschiedensten Einflüssen ausgesetzt. Neben mechanischen Beanspruchungen durch Bürsten, Kämmen, Hochstecken oder Zurückbinden werden die Haare auch durch Umwelteinflüsse wie zum Beispiel starke UV-Strahlung, Kälte, Wind und Wasser angegriffen. Auch der physiologische Status (z.B. Alter, Gesundheit) der jeweiligen Person beeinflusst den Zustand der keratinischen Fasern.

Insbesondere die Behandlung mit chemischen Mitteln verändert Struktur und Oberflächeneigenschaften der Haare. Methoden wie zum Beispiel das Dauerwellen, Bleichen, Färben, Tönen, Glätten usw., aber auch häufiges Waschen mit aggressiven Tensiden tragen dazu bei, dass mehr oder weniger starke Schäden an der Haarstruktur verursacht werden. So wird zum Beispiel bei einer Dauerwelle sowohl der Cortex als auch die Cuticula des Haares angegriffen. Die Disulfid-Brücken des Cystins werden durch den Reduktionsschritt aufgebrochen und im anschließenden Oxidationsschritt zum Teil zu Cysteinsäure oxidiert.

Beim Bleichen wird nicht nur das Melanin zerstört, sondern es werden außerdem ca. 15 bis 25% der Disulfid-Bindungen des Cystins bei einer milden Bleiche oxidiert. Bei einer exzessiven Bleichung können es sogar bis zu 45% sein (K. F. de Polo, A Short Textbook of Cosmetology, 2000, Verlag für chemische Industrie, H. Ziolkowsky GmbH).

So ergeben sich aus den chemischen Behandlungen, dem häufigen Waschen oder der UV-Bestrahlung nachteilige mechanische Eigenschaften für das Haar, hervorgerufen durch Entfernung natürlich ausgeschiedener Haarfette bzw. - feuchthaltemittel (Sebum). Es wird dadurch spröde, trocken, glanzlos, porös und schlecht kämmbar.

Außerdem ist gründlich gereinigtes Haar für gewöhnlich sehr schwer zu kämmen, sowohl in nassem als auch in trockenem Zustand, da die einzelnen Haare dazu neigen, kraus zu werden und sich zu verknoten. So verliert es erst beim Waschen und anschließend beim Kämmen seine Widerstandsfähigkeit. Dies zeigt sich in einer signifikanten Abnahme der Zug-Dehnungsfestigkeit bei nassem Haar. Außerdem ist es gegenüber einer weiteren Schädigung durch Chemikalien, Tenside und Umwelteinflüsse weniger widerstandsfähig als gesundes Haar.

Für die Pflege derart geschädigter Haare gibt es spezielle Zubereitungen, wie zum Beispiel Haarspülungen, Haarkuren, Shampoos, Leave-in Konditionierer usw., die jedoch vor allem die Kämmbarkeit, den Griff und den Glanz geschädigter Haare verbessern können. Derartige handelsübliche Haarpflegemittel enthalten hauptsächlich kationische Tenside auf Alkylammonium-Basis, Polymere, Wachse bzw. Öle oder Siliconöle. Die Wirksamkeit dieser Verbindungen lässt sich u.a. auf eine Hydrophobierung der Haar-Oberfläche zurückführen.

Bei allen diesen Mitteln wird zwar eine gute Pflegewirkung (Konditionierung) der Haare erreicht, aber das Aussehen, insbesondere der Glanz der Haare, wird durch die Pflegeprodukte nicht verbessert, sondern teilweise sogar verschlechtert.

Es besteht daher also weiterhin ein Bedarf an vielseitig einsetzbaren Wirkstoffen für Körperreinigungs- und Pflegemittel wie Shampoos, Haarbehandlungsmittel und Haarnachbehandlungsmittel, die neben der reinigenden Wirkung die Pflege des Haares verbessern und gleichzeitig guten Glanz verleihen, die das Haar vor der Schädigung der Haarstruktur schützen und die bereits verursachten strukturellen Schädigungen des Haares, hervorgerufen durch Umwelteinflüsse sowie form- und farbgebende Behandlungen, minimieren.

Es ist eine Aufgabe der Erfindung, einen solchen Wirkstoff zur Verfügung zu stellen, der in der Lage ist, sowohl Eigenschaften wie Kämmbarkeit, Weichheit, Volumen, Formbarkeit, Handhabbarkeit, die Entwirrbarkeit von ungeschädigten und geschädigten Haaren zu verbessern, als auch dem Haar einen schönen Glanz zu verleihen. Die Verbindungen sollen also eine verbesserte oder zumindest gleich gute Einzelwirkung, insgesamt aber eine verbesserte kombinierte Wirkung von mechanischen und anderen Eigenschaften zeigen.

Beschreibung der Erfindung:

Überraschenderweise wurde gefunden, dass quatäre Ammoniumgruppen enthaltende, im Siloxanteil verzweigte Siloxane der allgemeinen Formel (I), die gestellten Aufgaben lösen.

Ein Vorteil der erfindungsgemäßen Siloxane ist, dass die Vorteile von seitenständig modifizierten Siloxanen und von $\alpha,\omega$-modifizierten Siloxanen in ihnen vereint vorliegen und ein höherer Modifikationsgrad im Sinne einer größeren Anzahl an Substitutionsmöglichkeiten gegeben ist im Vergleich zu rein linearen Strukturen. Hierdurch sind Strukturen mit langem ungestörten Siloxanrückgrat zugänglich, die einen besonders guten konditionierenden Effekt in kosmetischen, dermatologischen und pharmazeutischen Formulierungen einzubringen vermögen.

Ein weiterer Vorteil der erfindungsgemäßen Siloxane ist, dass sie und die daraus gefertigten Folgeprodukte keine bzw. kaum Vergelungsneigung besitzen und somit über einen längeren Zeitraum gelagert werden können, ohne dass sich die Viskosität der Produkte maßgeblich verändert. Dieser Vorteil ist speziell bei hochmolekularen Produkten hervorzuheben. Die erfindungsgemäßen Produkte basieren somit vor allem auf quatäre Ammoniumgruppen enthaltenden, organomodifizierten, im Siliconteil verzweigten Siloxanen und damit hochverzweigten und auch höhermolekulareren (durchschnittliche Molmasse >3000 g/mol), dabei jedoch vergelungsfreien und damit vergleichsweise niedrigviskosen Strukturen.

Ein weiterer Vorteil der Erfindung ist, dass die Polysiloxane mit quatären Ammoniumgruppen gemäß Formel (I) hervorragende konditionierende Effekte auf Haut und Haare ausüben können. Durch diesen konditionierenden Effekt auf der Haut kann einem trockenen, spröden oder rauen Zustand der Haut nach Anwendungen einer wässrigen, tensidischen Formulierung vorgebeugt werden und ein angenehmes, samtigseidiges Hautgefühl erzielt wird.

Ein weiterer Vorteil ist, dass die erfindungsgemäße Verwendung als Pflegewirkstoff zu einem verbesserten Anschäumverhalten, einem erhöhten Schaumvolumen und einer besseren Schaumcremigkeit der Formulierungen beiträgt.

Zudem führt die erfindungsgemäße Verwendung der Strukturen als Pflegewirkstoff zu einem verbesserten Glanz von Haaren.

Werden im Rahmen der vorliegenden Erfindung Verbindungen, wie z. B. Polysiloxane, beschrieben, die verschiedene Einheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden Verbindungen zu verstehen.

Alle angegebenen Prozent (%) sind, wenn nicht anders angegeben, Massenprozent.

Alle Bedingungen wie beispielsweise Druck und Temperatur sind, wenn nicht anders angegeben, Standardbedingungen.

Gegenstand der vorliegenden Erfindung sind daher mindestens eine quatäre Ammoniumgruppen enthaltende Polysiloxane der allgemeinen Formel (I)

$$M_a\, M'_{a1}\, M''_{a2}\, M'''_{a3}\, D_b\, D'_{b1}\, D''_{b2}\, D'''_{b3}\, T_c\, T'_{c1}\, Q_d \qquad \text{Formel (I),}$$

wobei

M = $(R^1_3\ Si\ O_{1/2})$
M' = $(R^2R^1_2\ Si\ O_{1/2})$
M'' = $(R^3R^1_2\ Si\ O_{1/2})$
M''' = $(R^4R^1_2\ Si\ O_{1/2})$
D = $(R^1_2\ Si\ O_{2/2})$
D' = $(R^2R^1\ Si\ O_{2/2})$
D'' = $(R^3R^1\ Si\ O_{2/2})$D
D''' = $(R^4R^1\ Si\ O_{2/2})$

T = ($R^5$ Si $O_{3/2}$)
T' = ($R^2$ Si $O_{3/2}$)
Q = (Si $O_{4/2}$)

a = 0 bis 32; bevorzugt 0 bis 22, insbesondere 0 bis 12;
a1 = 0 bis 10, bevorzugt 0 bis 5, insbesondere 0;
a2 = 0 bis 32; bevorzugt 0 bis 22, insbesondere 1 bis 12;
a3 = 0 bis 10; bevorzugt 0 bis 5, insbesondere 0;

mit der Maßgabe, dass

$$a + a1 + a2 + a3 > 3, \text{ bevorzugt } > 4;$$

b = 1 bis 600, bevorzugt 10 bis 500, insbesondere 20 bis 400;
b1 = 0 bis 10, bevorzugt 0 bis 5, insbesondere 0;
b2 = 0 bis 80, bevorzugt 0 bis 50, insbesondere 0 bis 10;
b3 = 0 bis 20, bevorzugt 0 bis 10, insbesondere 0;

c = 0 bis 30, bevorzugt 1 bis 20, insbesondere 2 bis 15;
c1 = 0 bis 10, bevorzugt 0 bis 5, insbesondere 0;

d = 0 bis 15, bevorzugt 1 bis 12, insbesondere 2 bis 10;

mit der Maßgabe, dass

$$a2 + b2 > 3$$

und

$$c + c1 + d > 1, \text{ bevorzugt } > 2, \text{ insbesondere } \geq 3;$$

$R^1$ = unabhängig voneinander gleiche oder verschiedene lineare oder verzweigte, gegebenenfalls aromatische Kohlenwasserstoffreste mit 1 bis 30 Kohlenstoffatomen, bevorzugt Methyl oder Phenyl, insbesondere Methyl;

$R^2$ = unabgängig voneinander gleiche oder verschiedene Alkoxy- oder Acyloxyreste, wie beispielsweise Methoxy-, Ethoxy-, n-Propoxy oder iso-Propoxyreste, Acetoxy, insbesondere Ethoxy oder iso-Propoxyreste;

$R^3$ = unabhängig voneinander gleiche oder verschiedene organische Reste, die quatäre Ammoniumfunktionen tragen;

$R^4$ = unabhängig voneinander gleiche oder verschiedene organische Epoxy-Reste;

$R^5$ = unabhängig voneinander gleiche oder verschiedene Reste $R^1$, $R^3$ oder $R^4$, bevorzugt $R^1$, insbesondere Methyl, Phenyl, Dodecyl oder Hexadecyl

sind.

Geeignete Epoxy-Reste $R^4$ sind zum Beispiel vorzugsweise gleiche oder verschiedene Reste ausgewählt aus der Gruppe

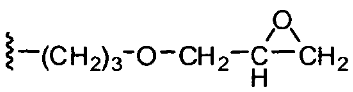

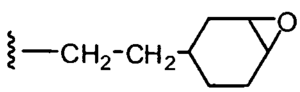

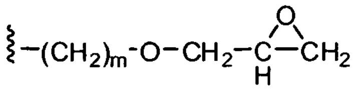

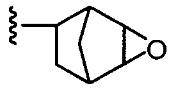

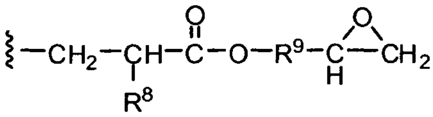

Geeignete Reste $R^3$ sind zum Beispiel Gruppen mit dem Aufbau

- $R^6$-$R^7$, worin

$R^6$ vorzugsweise gleiche oder verschiedene zweiwertige Reste sind, ausgewählt aus der Gruppe

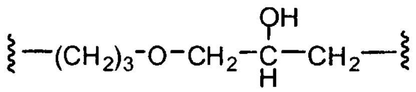

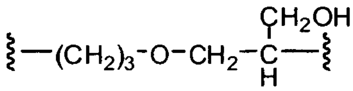

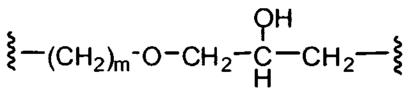

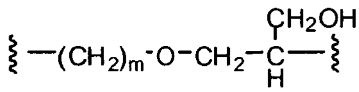

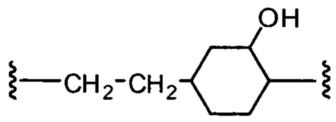

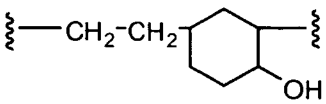

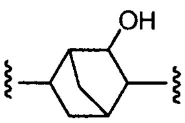

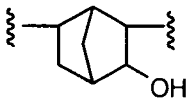

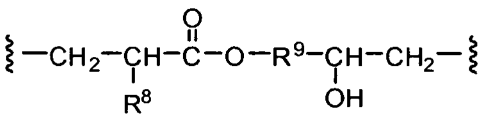

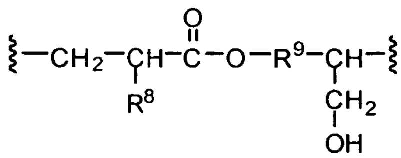

;

R" ist bevorzugt:

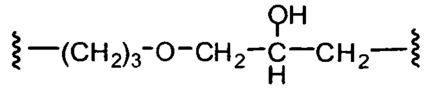

;

$R^7$ ausgewählt ist aus der Gruppe bestehend aus

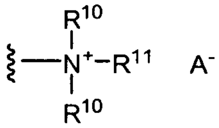

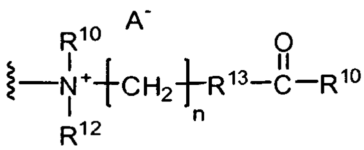

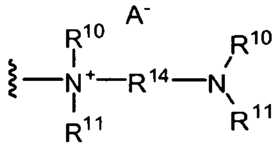 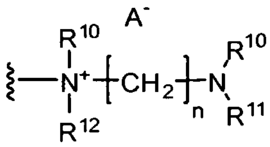 ;

| | |
|---|---|
| $R^8$ | sind gleiche oder verschiedene Reste aus der Gruppe Wasserstoff oder Alkyl mit 1 bis 6 C-Atomen, bevorzugt Methyl; |
| $R^9$ | sind gleiche oder verschiedene zweiwertige Kohlenwasserstoffreste, die gegebenenfalls Etherfunktionen enthalten, bevorzugt Methylen; |
| $R^{10}$, $R^{11}$, $R^{12}$ | sind jeweils unabhängig voneinander Wasserstoff oder Alkylreste mit 1 bis 30 C-Atomen oder Reste der Formel |

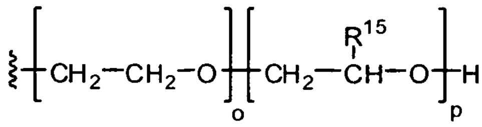 ;

| | |
|---|---|
| $R^{13}$ | sind gleiche oder verschiedene Reste aus der Gruppe -O-; $-NR^{16}-$; |
| $R^{14}$ | sind gleiche oder verschiedene gegebenenfalls verzweigte divalente Kohlenwasserstoffreste, bevorzugt Ethylen oder Propylen; |
| $R^{15}$ | sind gleiche oder verschiedene Alkyl-, Aryl- oder Alkarylreste mit 1 bis 30 C-Atomen, die gegebenenfalls Etherfunktionen enthalten, bevorzugt Methyl, Ethyl oder Phenyl, insbesondere Methyl; |
| $R^{16}$ | sind gleiche oder verschiedene Reste aus der Gruppe Wasserstoff oder Alkyl mit 1 bis 6 C-Atomen; |

m = 2 bis 18;
n = 2 bis 18, bevorzugt 3;
o = 0 bis 30, bevorzugt 0 bis 10, insbesondere 1 bis 3;
p = 0 bis 30, bevorzugt 0 bis 10;

$A^-$ sind gleiche oder verschiedene Gegenionen zu den positiven Ladungen an den quaternierten Stickstoffgruppen, ausgewählt aus anorganischen oder organischen Anionen der Säuren HA, sowie deren Derivate.

In einer weiteren bevorzugten Ausführungsform der vorliegenden Erfindung besteht das Gegenion $A^-$ zu den positiven Ladungen an den quaternierten Stickstoffgruppen aus dem Anion einer physiologisch verträglichen Säure HA, die besonders bevorzugt aus Essigsäure, L-Hydroxycarbonsäuren, insbesondere Milchsäure, oder aromatischen Carbonsäuren ausgewählt ist.

Weitere bevorzugte Gegenionen stammen aus gängigen Quaternierungsmitteln. Dies sind insbesondere Ethylsulfat, Methylsulfat, Toluolsulfonat, Chlorid und Bromid.

Es ist dem Fachmann geläufig, dass die Verbindungen in Form eines Gemisches mit einer im Wesentlichen durch statistische Gesetze geregelten Verteilung vorliegen.

Herstellung der erfindungsgemäßen Siloxane:

Ein weiterer Gegenstand der vorliegenden Erfindung ist ein Verfahren zur Herstellung der erfindungsgemäßen Polysiloxane der allgemeinen Formel (I) umfassend die Verfahrensschritte

A) Herstellung eines SiH-Gruppen-haltigen, über mindestens zwei Einheiten, ausgewählt aus T- und Q-Einheiten, verzweigten Siloxangrundgerüstes durch Equilibrierung und Kondensation einer Mischung, enthaltend die Komponenten

a) mindestens ein SiH-funktionelles Siloxan,

b) mindestens ein SiH-Funktion-freies Siloxan, und entweder Komponente c) oder Komponente d), oder beide Komponenten c) und d), wobei
c) mindestens ein Tetraalkoxysilan,
d) mindestens ein Trialkoxysilan unter Zugabe von Wasser und mindestens einem geeigneten Katalysator.

B) Hydrosilylierung der SiH-funktionellen Siloxane aus Verfahrensschritt A) mit mindestens einem ungesättigten Epoxid,
C) Quaternisierung der Epoxysiloxane aus Verfahrensschritt B) mit mindestens einem tertiären Amin.

Es ist erfindungsgemäß bevorzugt, dass der in Verfahrensschritt A) eingesetzte Katalysator ein fester, bevorzugt ein fester, Brönstedt-saurer Katalysator ist.

Als saure Ionenaustauscher können die nach dem Stand der Technik bekannten Ionenaustauscher eingesetzt werden. In dem erfindungsgemäßen Verfahren können in Verfahrensschritt A) sowohl natürliche Ionenaustauscher, wie beispielsweise Zeolithe, Montmorillonite, Attapulgite, Bentonite und andere Aluminiumsilikate sowie synthetische Ionenaustauscher eingesetzt werden. Letztere sind vorzugsweise Festkörper (meist in Körnerform) mit einer dreidimensionalen, wasserunlöslichen hochmolekularen Matrix auf der Basis von Phenol-Formaldehyd-Harzen oder Copolymerisate aus Styrol-Divinylbenzol, in die zahlreiche "Ankergruppen" unterschiedlicher Acidität eingebaut sind. Insbesondere können in Verfahrensschritt A) saure Tonerden oder saure Ionenaustauscherharze, wie beispielsweise die unter den Markennamen Amberlite®, Amberlyst® oder Dowex® und Lewatit® bekannten Produkte eingesetzt werden. Besonders bevorzugt wird als saurer Ionenaustauscher ein sulfonsaures Ionenaustauscherharz eingesetzt.

Als saure Ionenaustauscher werden in dem erfindungsgemäßen Verfahren in Verfahrensschritt A) vorzugsweise solche eingesetzt, wie sie in EP 1 439 200 beschrieben sind.

Es kann vorteilhaft sein, wenn in dem erfindungsgemäßen Verfahren in Verfahrensschritt A) als Katalysator mindestens ein fester saurer Ionenaustauscher (Katalysator 1) und mindestens ein weiterer, nicht fester Brönstedtsaurer Katalysator (Katalysator 2), insbesondere eine flüssige Säure eingesetzt wird. Als Katalysator 2 kann dabei eine Mineralsäure, vorzugsweise Schwefelsäure und/oder, vorzugsweise eine organische Sulfonsäure, vorzugsweise Trifluormethansulfonsäure eingesetzt werden. Diese Katalysatormischung wird vorzugsweise direkt der Reaktionsmischung zugegeben. Bevorzugt wird als Katalysator eine Mischung aus Trifluormethansulfonsäure und einem sulfonsauren Ionenaustauscherharz, vorzugsweise Lewatit® K 2621 (Bayer Material Science) eingesetzt. Bevorzugt weist die Katalysatormischung ein Massen-Verhältnis von Katalysator 1 zu Katalysator 2 von 10 zu 1 bis 100 zu 1 auf. Dieses Massenverhältnis ist insbesondere bei der Verwendung eines Lewatit®-Katalysators als Katalysator 1 und von Trifluormethansulfonsäure als Katalysator 2 bevorzugt.

Werden in Verfahrensschritt A) als Katalysator die zwei Katalysatoren 1 und 2 eingesetzt, so kann es vorteilhaft sein, wenn zu der Mischung an Ausgangsstoffen zunächst der Katalysator 2, vorzugsweise vollständig zugegeben wird, anschließend das Wasser zugefügt wird und erst nach der vorzugsweise vollständigen Zugabe von Wasser der Katalysator 1 zugegeben wird. Die Katalysatoren 1 und 2 können aber auch beide vor der Zugabe des Wassers den Ausgangsstoffen zugegeben werden.

Geeignete und bevorzugte Bedingungen für Verfahrensschritt A) sind insbesondere in den Patentanmeldungen DE 102008041601.0 und DE 102007055485.2 beschrieben, welche daher vollumfänglich als Teil der Offenbarung dieser Anmeldung zu betrachten sind.

Es ist in Verfahrensschritt A) möglich, dass je nach Art des Verfahrens restliche Alkoxygruppen (nach teilweise unvollständiger Kondensation) im SiH-funktionellen Siloxan enthalten sind. Dies kann z. B. dadurch erreicht werden, dass die Umsetzung vor Erreichen des vollständigen Umsatzes der Hydrolyse- und Kondensationsreaktion abgebrochen wird, oder dass das zur Hydrolyse benötigte Wasser in unterstöchiometrischen Verhältnissen eingesetzt wird, so dass nicht alle Alkoxygruppen der Alkoxysilane umgesetzt werden können.

Verfahrensschritt B) wird bevorzugt in Anwesenheit eines Edelmetallkatalysators, insbesondere Pt-, Rh- oder Ru-Katalysatoren, durchgeführt.

Bevorzugt in Verfahrensschritt B) eingesetzte, ungesättigte Epoxide sind zum Beispiel Allylglycidether, Vinylcyclohexenoxid, Norbornadienmonoepoxid. Geeignete und bevorzugte Bedingungen für die Hydrosilylierungsreaktion in Verfahrensschritt B) sind insbesondere z.B. in EP 1520870 beschrieben; diese wird hiermit als Referenz eingeführt und gilt als Teil des Offenbarungsgehaltes der vorliegenden Erfindung.

Es ist in Verfahrensschritt B) möglich, dass ein Teil der SiH-Gruppen nicht in einer SiC-Verknüpfungsreaktion abreagieren, sondern durch Reaktion an der Hydroxyfunktion SiOC-gebundene Alkoxy- oder Acyloxygruppen an das Siloxan geknüpft werden. Durch geeignete Wahl der Reaktionsbedingungen (v.a. Wahl des Katalysators/Cokatalysators, Reaktionstemperatur, Zugabesequenz der Reaktanden, Einsatz von Lösungsmitteln) kann diese Nebenreaktion meist hinreichend unterdrückt werden.

Die in Verfahrensschritt B) erhaltenen Epoxysiloxane können schließlich in Verfahrensschritt C) mit tertiären Aminen zu den gewünschten quatären ammoniumgruppen-tragenden Siloxanen umgesetzt werden. Geeignete und

bevorzugte Bedingungen für Verfahrensschritt C) sind beispielsweise in DE 37 19 086 und EP 0 294 642 beschrieben.

Es ist dem Fachmann bekannt, dass im Rahmen einer derartigen Reaktionssequenz sowohl bei der Equilibrierung der SiH-funktionellen Siloxane (Verfahrensschritt A), als auch bei der Hydrosilylierung (Verfahrensschritt B) und der Quaternisierung (Verfahrensschritt C) mit Nebenreaktionen zu rechnen ist. Der Umfang der Nebenreaktionen hängt unter anderem von der Art der Edukte als auch von den Reaktionsbedingungen ab. So liegt zum Beispiel der Quaternisierungsgrad bei der Umsetzung von Epoxysiloxanen mit tertiären Aminen in Gegenwart von Carbonsäuren nach gängigen Methoden bei etwa 80 bis 95%.

Es ist dem Fachmann offenbar, dass erfindungsgemäßes Verfahren zu Mischungen von Polysiloxanen führen wird, insbesondere zu technischen Mischungen. Unter dem im Zusammenhang mit der Erfindung verwendeten Begriff "gemäß dem erfindungsgemäßen Verfahren hergestellte Verbindung der allgemeinen Formel (I)" oder "gemäß dem erfindungsgemäßen Verfahren hergestellte Siloxan" auch solche Mischungen zu verstehen sind.

Verwendung der erfindungsgemäßen Produkte:

Ein weiterer Gegenstand dieser Erfindung ist die Verwendung des erfindungsgemäßen Polysiloxans oder eines nach dem erfindungsgemäßen Verfahren erhältlichen, bevorzugt erhaltenen, Polysiloxans zur Herstellung von kosmetischen, pharmazeutischen oder dermatologischen Kompositionen.

Es können erfindungsgemäß wasserlösliche oder wasserunlösliche Polysiloxane - auch für die im Folgenden genannten, erfindungsgemäßen Verwendungen - verwendet werden. Je nach zu erzeugender Formulierung (trübe oder klare Formulierungen) ist es dem Fachmann geläufig, ob wasserlösliche oder unlösliche Polysiloxane zur Herstellung der Formulierung eingesetzt werden sollten. Der Begriff "wasserunlöslich" im Sinne der vorliegenden Erfindung ist definiert als eine Löslichkeit von weniger als 0.01 Gewichtsprozent in wässriger Lösung bei 20 °C und 1 bar Druck. Der Begriff "wasserlöslich" im Sinne der vorliegenden Erfindung ist definiert als eine Löslichkeit von gleich oder mehr als 0.01 Gewichtsprozent in wässriger Lösung bei 20 °C und 1 bar Druck.

Ein weiterer Gegenstand dieser Erfindung ist die Verwendung des erfindungsgemäßen Polysiloxans oder eines nach dem erfindungsgemäßen Verfahren erhältlichen, bevorzugt erhaltenen, Polysiloxans als Pflegewirkstoff in, bevorzugt tensidhaltigen wässrigen, Pflege- und Reinigungsformulierungen.

Unter dem Begriff "Pflegewirkstoff" wird hier eine Substanz verstanden, die den Zweck erfüllt, einen Gegenstand in seiner ursprünglichen Form zu erhalten, die Auswirkungen äußerer Einflüsse (z.B. Zeit, Licht, Temperatur, Druck, Verschmutzung, chemische Reaktion mit anderen, mit dem Gegenstand in Kontakt tretenden reaktiven Verbindungen) wie beispielsweise Altern, Verschmutzen, Materialermüdung, Ausbleichen, zu mindern oder zu vermeiden oder sogar gewünschte positive Eigenschaften des Gegenstandes zu verbessern. Für letzten Punkt sei etwa ein verbesserter Haarglanz oder eine größere Elastizität des betrachteten Gegenstandes genannt.

Eine bevorzugte Pflegeformulierung ist in diesem Zusammenhang eine glanzverbessernde Pflegeformulierung.

In diesem Zusammenhang sind die Pflege- und Reinigungsformulierungen nicht auf kosmetische, pharmazeutische oder dermatologische Kompositionen beschränkt, sondern können auch solche Formulierungen sein, wie man in Haushalt und Industrie einsetzt, etwa zur Pflege und Reinigung von Oberflächen nicht lebender Gegenstände wie beispielsweise Fliesen, Holz, Glas, Keramik, Linoleum, Kunststoff, lackierte Oberflächen, Leder, Stoffe, Fasern. Beispiele solcher Gegenstände sind Fensterscheiben und - bänke, Duschabtrennungen, Fußböden wie Teppiche, Fliesen, Laminate, Parkett, Korkfußböden, Marmor-, Stein- und Feinsteinzeugböden Haushaltskeramiken wie WCs, Waschbecken, Bidets, Duschtassen, Badewannen, Türklinken, Armaturen, Haushaltswerkzeuge wie Waschmaschinen, Trockner, Spülmaschinen, Spülen aus Keramik oder Edelstahl, Möbel wie Tische, Stühle, Regale, Ablageflächen, Fenster, Kochgeschirr, Geschirr und Besteck, Wäsche, insbesondere körpernahe Wäsche ("Unterwäsche"),Wasserfahr-, Fahr- und Flugzeuge wie Autos, Busse, Motor- und Segelboote Werkzeuge wie chirurgische Instrumente, Staubsauger, Maschinen, Rohrleitungen, Tanks und Geräte für Transport, Verarbeitung und Aufbewahrung in der Lebensmittelverarbeitung. Somit handelt es sich in diesem Zusammenhang um die Verwendung in Reinigungs- und Pflegemitteln für Haushalt, industrielle und institutionelle Gewerbe.

In diesem Zusammenhang handelt es sich bei der zu pfelgenden und reinigenden Oberfläche bevorzgt um die einer Faser oder eines Textils, insbesondere um die Oberfläche von gewobenen Textilien, Wäsche, Polstern oder Teppichen. Ein weiterer Gegenstand dieser Erfindung ist die Verwendung des erfindungsgemäßen Polysiloxans oder eines nach dem erfindungsgemäßen Verfahren erhältlichen, bevorzugt erhaltenen, Polysiloxans als Konditioniermittel für Haarbehandlungsmittel und Haarnachbehandlungsmittel sowie als Mittel zur Verbesserung der Haarstruktur.

Formulierungen/Kompositionen

Ein weiterer Gegenstand dieser Erfindung sind kosmetische, pharmazeutische oder dermatologische Kompositionen, insbesondere bevorzugt tensidhaltige wässrige Pflege- und Reinigungsformulierungen, insbesondere Haarbehandlungsmittel und Haarnachbehandlungsmittel zum Ausspülen oder zum Verbleib im Haar, beispielsweise Shampoos

mit oder ohne ausgeprägter Konditionierwirkung, 2in1-Shampoos, Spülungen, Haarkuren, Haarmasken, Frisierhilfen, Stylingmittel, Fönlotionen, Haarfestiger, Dauerwellmittel, Haarglättungsmittel und Mittel zum Färben der Haare enthaltend mindestens eines der erfindungsgemäßen Polysiloxane oder eines der nach dem erfindungsgemäßen Verfahren erhältlichen, bevorzugt erhaltenen, Polysiloxane. Die erfindungsgemäßen Polysiloxane werden vorteilhaft in einer Konzentration von 0.01 bis 20 Massenprozent, bevorzugt 0.1 bis 8 Massenprozent, besonders bevorzugt von 0.2 bis 4 Massenprozent in den erfindungsgemäßen Kompositionen eingesetzt.

Die erfindungsgemäße Komposition kann z.B. mindestens eine zusätzliche Komponente enthalten, ausgewählt aus der Gruppe der

Emollients,
Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.

Bezüglich weiterer fakultativer Komponenten sowie die eingesetzten Mengen dieser Komponenten wird ausdrücklich auf die dem Fachmann bekannten einschlägigen Handbücher, z. B. K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung.
Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Ein weiterer Gegenstand dieser Erfindung sind Reinigungs- und Pflegeformulierungen für Haushalt, industrielle und institutionelle Anwendungen wie beispielsweise Desinfektionsmittel, Desinfektionsreiniger, Schaumreiniger, Fußbodenreiniger, Teppichreiniger, Polsterreiniger, Fußbodenpflegeprodukte, Marmorreiniger, Parkettreiniger, Stein- und Keramikbodenreiniger, Wischpflegemittel, Edelstahlreiniger, Glasreiniger, Geschirrspülmittel, Kunststoffreiniger, Sanitärreiniger, Holzreiniger, Lederreiniger, Waschmittel, Wäschepflegemittel Desinfektionswaschmittel, Vollwaschmittel, Feinwaschmittel, Wollwaschmittel, Weichspülmittel, Imprägniermittel enthaltend mindestens eines der erfindungsgemäßen Polysiloxane oder eines der nach dem erfindungsgemäßen Verfahren erhältlichen, bevorzugt erhaltenen, Polysiloxane. In diesem Zusammenhang bevorzugte Reinigungs- und Pflegeformulierungen für Haushalt, industrielle und institutionelle Anwendungen sind Waschmittel, Wäschepflegemittel, Vollwaschmittel, Feinwaschmittel, Wollwaschmittel, Weichspülmittel, Imprägniermittel, insbesondere Weichspülmittel.

Ausführungsbeispiele:

In den nachfolgend aufgeführten Beispielen wird die vorliegende Erfindung zur Verdeutlichung der Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten

werden können. Werden im Rahmen der vorliegenden Beschreibung Dokumente zitiert, so soll deren Inhalt vollständig zum Offenbarungsgehalt der vorliegenden Erfindung gehören. Werden im Rahmen der vorliegenden Erfindung Verbindungen, wie z.B. organomodifizierte Polysiloxane, beschrieben, die verschiedene Monomereinheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als statistische Mittelwert, gemittelt über alle entsprechenden Verbindungen, zu verstehen.

<u>Herstellung des erfindungsgemäßen Beispielproduktes Nr. 1:</u>

A) Equilibrierung eines verzweigten SiH-funktionellen Polysiloxans

Nach Vorschrift der Patentanmeldungen DE 102008041601.0 und DE 102007055485.2 wurden 22,9 g (0,11 mol) Tetraethoxysilan (>98%, erhältlich bei der Firma Fluka), 366,4 g (0,99 mol) Decamethylcyclopentasiloxan (erhältlich bei der Firma Gelest Inc.) und 112,6 g eines $\alpha,\omega$-Dihydrogenpolydimethylsiloxans mit einem Wasserstoffgehalt (SiH) von 2,93 mol SiH/kg in einem Vierhalskolben, ausgestattet mit KPG-Rührer, Innenthermometer, Tropftrichter und Destillationsbrücke unter Rühren bei 40 °C vorgelegt. 0,5 g Trifluormethansulfonsäure (erhältlich bei Sigma Aldrich) wurden zugegeben und es wurde 2 h gerührt. Anschießend wurde innerhalb von 5 min unter Rühren eine Mischung aus 7,9 g deionisiertem Wasser und 2,0 g Ethanol zugetropft und 1 h bei 40 °C gerührt. Nach Zugabe von 30,1 g des vorgetrockneten sulfonsauren Kationenaustauscherharzes Lewatit® K 2621 (10 Gew% Wassergehalt - bestimmt in Anlehnung an die Karl-Fischer-Methode) wurde überschüssiges Wasser und Alkohol im Vakuum von ca. 15 mbar für 1 h bei 40 °C abdestilliert. Nach Abfiltrieren des Harzes wurde mit 10,0 g Natriumhydrogencarbonat neutralisiert und erneut filtriert. Man erhielt eine klare, farblose Flüssigkeit mit einem Wasserstoff(SiH)-Gehalt von 0,0655%.

B) Herstellung eines Epoxysiloxans

In einem 500 ml Dreihalskolben versehen mit KPG-Rührer, Tropftrichter, Innenthermometer und Rückflußkühler wurden 230,8 g (0,15 mol SiH) des gemäß Beispiel 1a) hergestellten SiH-Siloxans mit 22,3 g (0,20 mol) Allylglycidylether unter Zusatz von 15 ppm cis-Platin-Katalysator bei 120 °C unter Stickstoffatmosphäre umgesetzt. Nach 2 h wurde vollständiger SiH-Umsatz erreicht. Durch anschließende Destillation bei 120 °C und 1 mbar erhielt man eine klare, farblose Flüssigkeit mit einem Epoxygehalt von 0,98%.

C) Umsetzung zum quatären Polysiloxan

In einem 500 ml Dreihalskolben versehen mit KPG-Rührer, Tropftrichter, Innenthermometer und Rückflußkühler wurden 27,0 g (0,095 mol) 3-N,N-Dimethyl-aminopropyl-laurylsäureamid, 5,9 g (0,098 mol) Essigsäure und 80 ml Isopropanol bei Raumtemperatur vorgelegt und für 1 h gerührt. Anschließend wurden 155,1 g (0,095 mol Epoxy) der gemäß Beispiel 1b) hergestellten Verbindung zugetropft. Anschließend wurde 8 h bei 65 °C unter Stickstoffatmosphäre gerührt. Das Isopropanol wurde schließlich bei 65 °C und 1 mbar abdestilliert. Es wurde eine klare, gelbliche, hochviskose Flüssigkeit erhalten, welche durch die folgende statistische Formel beschrieben wird:

$$(R^2Me_2SiO_{1/2})_6\ (Me_2SiO_{1/2})_{112}\ (SiO_{4/2})_2$$

mit $R^2$ =

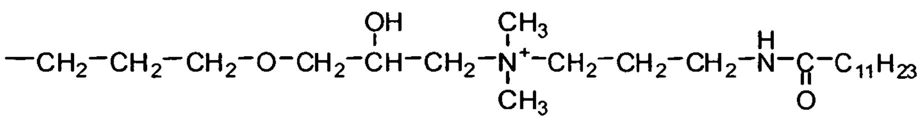

$CH_3COO^-$

<u>Herstellung des erfindungsgemäßen Beispielproduktes Nr. 2:</u>

A) Equilibrierung eines verzweigten SiH-funktionellen Polysiloxans

Nach Vorschrift der Patentanmeldungen DE 102008041601.0 und DE 102007055485.2 wurden 89,2 g (0,50 mol) Methyltriethoxysilan (Dynasylan® MTES der Firma Evonik Degussa GmbH), 1023,4 g (2,76 mol) Decamethylcyclopentasiloxan (erhältlich bei der Firma Gelest Inc.) und 47,0 g (0,35 mol) 1,1,3,3-Tetramethyldisiloxan (erhältlich bei

der Firma Gelest Inc.) in einem Vierhalskolben, ausgestattet mit KPG-Rührer, Innenthermometer, Tropftrichter und Destillationsbrücke unter Rühren bei 40 °C vorgelegt. 1,2 g Trifluormethansulfonsäure (erhältlich bei Sigma Aldrich) wurden zugegeben und es wurde 2 h gerührt. Anschießend wurde innerhalb von 5 min unter Rühren eine Mischung aus 27,0 g deionisiertem Wasser und 6,8 g Ethanol zugetropft und 1 h bei 40 °C gerührt. Nach Zugabe von 70,0 g des vorgetrockneten sulfonsauren Kationenaustauscherharzes Lewatit® K 2621 (10 Gew% Wassergehalt - bestimmt in Anlehnung an die Karl-Fischer-Methode) wurde überschüssiges Wasser und Alkohol im Vakuum von ca. 15 mbar für 1 h bei 40 °C abdestilliert. Nach Abfiltrieren des Harzes wurde mit 23,2 g Natriumhydrogencarbonat neutralisiert und erneut filtriert. Man erhielt eine klare, farblose Flüssigkeit mit einem Wasserstoff(SiH)-Gehalt von 0,0634%.

B) Herstellung eines Epoxysiloxans

In einem 500 ml Dreihalskolben versehen mit KPG-Rührer, Tropftrichter, Innenthermometer und Rückflußkühler wurden 200,0 g (0,126 mol SiH) des gemäß Beispiel 2a) hergestellten SiH-Siloxans mit 18,7 g (0,164 mol) Allylglycidylether unter Zusatz von 15 ppm cis-Platin-Katalysator bei 120 °C unter Stickstoffatmosphäre umgesetzt. Nach 2 h wurde vollständiger SiH-Umsatz erreicht. Durch anschließende Destillation bei 120 °C und 1 mbar erhielt man eine klare, farblose Flüssigkeit mit einem Epoxygehalt von 0,95%.

C) Umsetzung zum quatären Polysiloxan

In einem 500 ml Dreihalskolben versehen mit KPG-Rührer, Tropftrichter, Innenthermometer und Rückflußkühler wurden 25,3 g (0,089 mol) 3-N,N-Dimethyl-aminopropyl-laurylsäureamid, 5,5 g (0,092 mol) Essigsäure und 80 ml Isopropanol bei Raumtemperatur vorgelegt und für 1 h gerührt. Anschließend wurden 150,0 g (0,089 mol Epoxy) der gemäß Beispiel 2b) hergestellten Verbindung zugetropft. Anschließend wurde 8 h bei 65 °C unter Stickstoffatmosphäre gerührt. Das Isopropanol wurde schließlich bei 65 °C und 1 mbar abdestilliert. Es wurde eine klare, gelbliche, hochviskose Flüssigkeit erhalten, welche durch die folgende statistische Formel beschrieben wird:

$$(R^2Me_2SiO_{1/2})_7\ (Me_2SiO_{1/2})_{138}\ (MeSiO_{3/2})_5$$

mit $R^2$ =

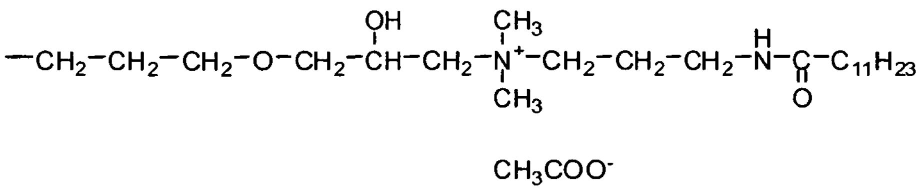

Herstellung des Vergleichsbeispielproduktes Nr. 3 (nicht erfindungsgemäß) :

a) Herstellung eines Epoxysiloxans

In einem 1 l Dreihalskolben versehen mit KPG-Rührer, Tropftrichter, Innenthermometer und Rückflußkühler wurden 367 g (0,4 mol SiH) eines klassisch kammständig SiH-modifizierten Siloxans der Formel

$$(Me_3SiO_{1/2})_2\ (Me_2SiO_{1/2})_{44}\ (MeHSiO_{1/2})_4$$

mit 59 g (0,52 mol) Allylglycidylether unter Zusatz von 15 ppm cis-Platin-Katalysator bei 120 °C unter Stickstoffatmosphäre umgesetzt. Nach 2 h wurde vollständiger SiH-Umsatz erreicht. Durch anschließende Destillation bei 120 °C und 1 mbar erhielt man eine klare, farblose Flüssigkeit mit einem Epoxygehalt von 1,55%.

b) Umsetzung zum quatären Polysiloxan

In einem 500 ml Dreihalskolben versehen mit KPG-Rührer, Tropftrichter, Innenthermometer und Rückflußkühler wurden 28,4 g (0,10 mol) 3-N,N-Dimethyl-aminopropyl-laurylsäureamid, 6,3 g (0,105 mol) Essigsäure und 120 ml Isopropanol bei Raumtemperatur vorgelegt und für 1 h gerührt. Anschließend wurden 206 g (0,1 mol Epoxy) der gemäß Beispiel 3a) hergestellten Verbindung zugetropft. Anschließend wurde 8 h bei 65 °C unter Stickstoffatmosphäre gerührt. Das Isopropanol wurde schließlich bei 65 °C und 1 mbar abdestilliert. Es wurde eine klare, gelbliche, hochviskose Flüssigkeit erhalten, welche durch die folgende statistische Formel beschrieben wird:

$(Me_3SiO_{1/2})_2 (Me_2SiO_{1/2})_{44} (RMeSiO_{1/2})_4$

mit R =

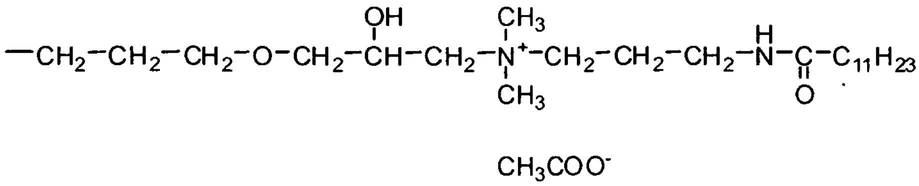

Anwendungstechnische Eigenschaften

Die Formulierungsbestandteile sind in den Zusammensetzungen in Form der allgemein anerkannten INCI-Nomenklatur unter Verwendung der englischen Begriffe benannt. Alle Konzentrationen in den Anwendungsbeispielen sind in Gewichtsprozent angegeben.

1.) Austestung der Konditionierung von Haut (Hautpflegeleistung) und Schaumeigenschaften mittels eines Handwaschtests:

Zur Bewertung der Konditionierung von Haut (Hautpflegeleistung) und der Schaumeigenschaften des erfindungsgemäßen organomodifizierten, im Siliconteil verzweigten Siloxans Produkt Nr. 1 in wäßrigen, tensidischen Formulierungen wurden sensorische Handwaschtests im Vergleich zum Vergleichsbeispiel 3 nach dem Stand der Technik durchgeführt.

Das Vergleichsbeispiel 3 ist in der Industrie als Pflegewirkstoffe weit verbreitet und gilt als hochwirksamer Pflegewirkstoff in wässrigen, tensidischen Formulierungen. Eine Gruppe bestehend aus 10 trainierten Prüfpersonen wusch sich dabei definiert die Hände und bewertete Schaumeigenschaften und Hautgefühl anhand einer Notenskala von 1 (schlecht) bis 5 (sehr gut).

Die eingesetzten Produkte wurden jeweils in einer standardisierten Tensidformulierung (Tabelle 1) getestet. Als Kontrollformulierung 0b wird eine Formulierung ohne Zusatz eines organomodifizierten Siloxans verwendet.

Tab.1: Testformulierungen für Handwaschtest.

| Formulierungsbeispiele | 0b | 1b | V2b |
|---|---|---|---|
| Texapon NSO®, 28%-ig, Cognis (INCI: Sodium Laureth Sulfate) | 32% | 32% | 32% |
| TEGO Betain F 50®, 38%-ig, Evonik Goldschmidt GmbH (INCI: Cocamidopropyl Betaine) | 8% | 8% | 8% |
| NaCl | 2% | 2% | 2% |
| Wasser, demineralisiert | ad 100,0% | | |
| Produkt Nr.1 (erfindungsgemäß) | | 0,5% | |
| Produkt Nr. 3 (nicht erfindungsgemäß) | | | 0,5% |

Die sensorischen Testergebnisse sind in Tabelle 2 zusammengefasst.

| Testformulierung | 0b | 1b | V2b |
|---|---|---|---|
| Anschäumverhalten | 3,0 | 3,5 | 3,3 |
| Schaumvolumen | 2,8 | 3,2 | 2,9 |
| Schaumcremigkeit | 2,3 | 3,3 | 3,0 |
| Hautgefühl während des Waschens | 2,8 | 3,8 | 3,7 |
| Hautglätte | 1,4 | 3,3 | 2,9 |
| Hautweichheit | 2,0 | 3,1 | 2,9 |

(fortgesetzt)

| Testformulierung | 0b | 1b | V2b |
|---|---|---|---|
| Hautglätte nach 3 min. | 2,6 | 3,9 | 3,6 |
| Hautweichheit nach 3 min. | 2,5 | 3,8 | 3,5 |
| Tab.2: Ergebnisse des Handwaschtests | | | |

In Tabelle 2 sind die Ergebnisse des Handwaschtests dargestellt. Anhand der Messergebnisse wird ersichtlich, dass die erfindungsgemäße Formulierung 1b unter Verwendung des erfindungsgemäßen Produkts Nr. 1 in allen Applikationseigenschaften im Vergleich zur Vergleichsformulierung V2b nach dem Stand der Technik überlegen ist.

Vor diesem Hintergrund sind die Ergebnisse der erfindungsgemäßen Formulierung 1b als sehr gut zu bezeichnen.

Anhand der Messwerte ist ersichtlich, dass das erfindungsgemäße Produkt Nr.1 in der Formulierung 1b zu einer Verbesserung der Hauteigenschaften und Schaumeigenschaften im Vergleich zum Produkt Nr. 3 in der Formulierung V2b führt.

Ferner kann man den Meßwerten entnehmen, dass die Kontrollformulierung 0b ohne eine Siliconverbindung schlechtere Meßwerte als die Formulierungen 1b und V2b aufweist.

2.) Austestung der Konditionierung von Haar mittels

Sensoriktests:

Für die anwendungstechnische Beurteilung der Konditionierung von Haar wurden das erfindungsgemäße Produkt Nr. 2 und das Vergleichsprodukt 3 in einfachen kosmetischen Formulierungen (Shampoo und Haarspülung) eingesetzt.

Die Anwendungseigenschaften beim Einsatz in einem Shampoo wurden in den folgenden Rezepturen überprüft:

Tab.3: Shampooformulierungen zur Austestung der haarkonditionierenden Eigenschaften.

| Formulierungsbeispiele | 0c | 1c | V2c |
|---|---|---|---|
| Texapon NSO®, 28%-ig, Cognis (INCI: Sodium Laureth Sulfate) | 32% | 32% | 32% |
| TEGO© Betain F 50, 38%-ig, Evonik Goldschmidt GmbH (INCI: Cocamidopropyl Betaine) | 8% | 8% | 8% |
| Jaguar 162, Rhodia (INCI: Guar Hydroxypropyl trimonium Chloride) (Kationisches Polymer zur Verbesserung der Wirksamkeit von Konditioniermitteln) | 0,3% | 0,3% | 0,3% |
| Wasser, demineralisiert | ad 100,0% | | |
| Zitronensäure | ad. pH 6,0 $\pm$ 0,3 | | |
| Produkt Nr. 2 (erfindungsgemäß) | | 0,5% | |
| Produkt Nr. 3 (nicht erfindungsgemäß) | | | 0,5% |

Zur Bewertung der Eigenschaften der Shampooformulierung wurde im Testablauf keine Nachbehandlung mit einer Spülung durchgeführt.

Die Anwendungseigenschaften beim Einsatz in Haarspülungen wurden in den folgenden Rezepturen überprüft:

| Formulierungsbeispiele | 0d | 1d | V2d |
|---|---|---|---|
| TEGINACID®C, Evonik Goldschmidt GmbH (INCI: Ceteareth-25) | 0,5% | 0,5% | 0,5% |
| TEGO®Alkanol 16, Evonik Goldschmidt GmbH (INCI: Cetyl Alcohol)) | 4% | 4% | 4% |
| VARISOFT® 300, 30%-ig, Evonik Goldschmidt GmbH (INCI: Cetrimonium Chloride) | 3,3% | 3,3% | 3,3% |
| Wasser, demineralisiert | Ad 100,0% | | |
| Zitronensäure | ad. pH 4,0 $\pm$ 0,3 | | |

(fortgesetzt)

| Formulierungsbeispiele | 0d | 1d | V2d |
|---|---|---|---|
| Produkt Nr. 2 (erfindungsgemäß) | | 0,5% | |
| Produkt Nr. 3 (nicht erfindungsgemäß) | | | 0,5% |
| Tab.4: Haarspülung Formulierungen zur Austestung der haarkonditionierenden Eigenschaften. | | | |

Die Vorbehandlung der Haare erfolgt im Falle der Eigenschaftsprüfung von Haarspülungen durch ein Shampoo, welches keine Konditioniermittel enthält.

Für die anwendungstechnische Beurteilung werden Haartressen, die für sensorische Tests verwendet werden, durch eine Dauerwellbehandlung und eine Bleichbehandlung standardisiert vorgeschädigt. Dazu werden friseurübliche Produkte verwendet. Der Testablauf, die verwendeten Basismaterialien sowie die Details der Beurteilungskriterien sind in DE 103 27 871 beschrieben.

Standardisierte Behandlung von vorgeschädigten Haarsträhnen mit konditionierenden Proben:

Die, wie oben beschrieben, vorgeschädigten Haarsträhnchen werden wie folgt mit dem oben beschriebenen Shampoo oder der oben beschriebenen konditionierenden Spülung behandelt:

Die Haarsträhnen werden unter fließendem, warmem Wasser benetzt. Das überschüssige Wasser wird leicht von Hand ausgedrückt, dann wird das Shampoo aufgebracht und sanft im Haar eingearbeitet (1 ml/Haarsträhne (2 g)). Nach einer Verweilzeit von 1 min wird das Haar für 1 min gespült.
Gegebenenfalls wird direkt im Anschluss die Spülung aufgebracht und sanft im Haar eingearbeitet (1 ml/Haarsträhne (2 g)). Nach einer Verweilzeit von 1 min wird das Haar für 1 min gespült.

Vor der sensorischen Beurteilung wird das Haar an der Luft bei 50% Luftfeuchtigkeit und 25 °C für mindestens 12 h getrocknet.

Beurteilungskriterien:

Die sensorischen Bewertungen erfolgen nach Noten, die auf einer Skala von 1 bis 5 vergeben werden, wobei 1 die schlechteste und 5 die beste Bewertung ist. Die einzelnen Testkriterien erhalten jeweils eine eigene Bewertung.

Die Testkriterien sind: Nasskämmbarkeit, Nassgriff, Trockenkämmbarkeit, Trockengriff, Aussehen/Glanz.

In der folgenden Tabelle werden die Ergebnisse der sensorischen Beurteilung der wie oben beschrieben durchgeführten Behandlung der Haarstränchen mit der erfindungsgemäßen Formulierung 1c, der Vergleichsformulierung V2c und der Kontrollformulierung 0c (Placebo ohne Testsubstanz) verglichen.

Tab.5: Ergebnisse der Konditionierung von Haar aus ShampooFormulierung

| | Nasskämmbarkeit | Nassgriff | Trockenkämmbarkeit | Trockengriff | Glanz |
|---|---|---|---|---|---|
| Erfindungsgemäße Formulierung 1c | 3,7 | 3,5 | 3,3 | 4,3 | 4,1 |
| Vergleichsformulierung V2c (nicht erfindungsgemäß) | 3,2 | 3,1 | 3,1 | 3,8 | 3,3 |
| Kontrollformulierung 0c (Placebo) | 2,3 | 2,5 | 2,5 | 3,3 | 2,3 |

Die Ergebnisse zeigen in überraschender Weise, dass die erfindungsgemäße Formulierung 1c mit erfindungsgemäßem Produkt Nr. 2 signifikant bessere Bewertungen erhält, als die Vergleichsformulierung V2c mit dem Produkt 3 nach Stand der Technik. Besonders deutlich ist die gute Bewertung der Glanzeigenschaften aller erfindungsgemäßen Formulierungen hervorzuheben.

Tab.6: Ergebnisse der Konditionierung von Haar aus Haarspülung-Formulierungen

| | Nasskämmbarkeit | Nassgriff | Trockenkämmbarkeit | Trockengriff | Glanz |
|---|---|---|---|---|---|
| Erfindungsgemäße Formulierung 1d | 4, 9 | 4,9 | 4,7 | 4,8 | 4,5 |
| Vergleichsformulierung V2d(nicht erfindungsgemäß) | 4,4 | 4,3 | 4,4 | 4,5 | 3,9 |
| Kontrollformulierung 0d | 3,8 | 3,9 | 4,0 | 3,8 | 2,9 |

Auch in der Anwendung Haarspülung zeigt die erfindungsgemäße Formulierung 1d mit erfindungsgemäßem Produkt Nr. 2 in der sensorischen Beurteilung sehr gute kosmetische Bewertungen. Dabei wurden die bereits sehr guten Eigenschaften der Vergleichsformulierung V2d mit dem Vergleichsprodukt Nr. 3 durch die erfindungsgemäße Formulierung 1d mit der erfindungsgemäßen Verbindung Nr.2 noch weiter gesteigert.

Eine signifikant bessere Bewertung wird auch beim Glanz durch die Verwendung der erfindungsgemäßen Formulierung 1d erreicht.

Formulierungsbeispiele:

Die folgenden Formulierungsbeispiele zeigen, dass erfindungsgemäßen Polysiloxane mit quatären Ammoniumgruppen in einer Vielzahl kosmetischer Formulierungen eingesetzt werden können.

Formulierungsbeispiel 1) Clear Shampoo

| | |
|---|---|
| TEXAPON® NSO, Cognis, 28%-ig (INCI: Sodium Laureth Sulfate) | 32,00% |
| Verbindung Beispiel 1 | 0,50% |
| Perfume | 0,50% |
| Water | 57,50% |
| TEGO® Betain F 50, Evonik Goldschmidt GmbH, 38%-ig (INCI: Cocamidopropyl Betaine) | 8,00% |
| ANTIL® 171 Evonik Goldschmidt GmbH (INCI: PEG-18 Glyceryl Oleate/Cocoate) | 1,00% |
| NaCl | 0,50% |
| Preservative | q.s. |

Formulierungsbeispiel 2) Clear Conditioning Shampoo

| | |
|---|---|
| TEXAPON® NSO, Cognis, 28%-ig (INCI: Sodium Laureth Sulfate) | 32,00% |
| Verbindung Beispiel 1 | 1,00% |
| Perfume | 0,50% |
| Water | 55,70% |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,30% |
| TEGO® Betain F 50, Evonik Goldschmidt GmbH, 38%-ig (INCI: Cocamidopropyl Betaine) | 8,00% |
| NaCl | 1,50% |
| Preservative | q.s. |

Formulierungsbeispiel 3) Clear Conditioning Shampoo

| | |
|---|---|
| TEXAPON® NSO, Cognis, 28%-ig (INCI: Sodium Laureth Sulfate) | 32,00% |

(fortgesetzt)

| | |
|---|---|
| ANTIL® 200, Evonik Goldschmidt GmbH (INCI: PEG-200 Hydrogenated Glyceryl Palmate; PEG-7 Glyceryl Cocoate) | 2,00% |
| Verbindung Beispiel 2 | 1,00% |
| Perfume | 0,25% |
| Water | 56,25% |
| Polymer JR 400, Amerchol (INCI: Polyquaternium-10) | 0,20% |
| TEGO® Betain F 50, Evonik Goldschmidt GmbH, 38%-ig (INCI: Cocamidopropyl Betaine) | 8,00% |
| NaCl | 0,30% |
| Preservative | q.s. |

Formulierungsbeispiel 4) Clear Conditioning Shampoo

| | |
|---|---|
| TEXAPON® NSO, Cognis, 28%-ig (INCI: Sodium Laureth Sulfate) | 32,00% |
| ANTIL® 200, Evonik Goldschmidt GmbH (INCI: PEG-200 Hydrogenated Glyceryl Palmate; PEG-7 Glyceryl Cocoate) | 2,00% |
| ABIL® Quat 3272, Evonik Goldschmidt GmbH (INCI: Quaternium-80) | 0,75% |
| Verbindung Beispiel 1 | 0,50% |
| Perfume | 0,25% |
| Water | 56,00% |
| Polymer JR 400, Amerchol (INCI: Polyquaternium-10) | 0,20% |
| TEGO® Betain F 50, Evonik Goldschmidt GmbH, 38%-ig (INCI: Cocamidopropyl Betaine) | 8,00% |
| NaCl | 0,30% |
| Preservative | q.s. |

Formulierungsbeispiel 5) Clear Conditioning Shampoo

| | |
|---|---|
| TEXAPON® NSO, Cognis, 28%-ig (INCI: Sodium Laureth Sulfate) | 32,00% |
| ANTIL® 200, Evonik Goldschmidt GmbH (INCI: PEG-200 Hydrogenated Glyceryl Palmate; PEG-7 Glyceryl Cocoate) | 2,00% |
| ABIL® B 8832, Evonik Goldschmidt GmbH (INCI: Bis-PEG/PPG-20/20 Dimethicone) | 1,00% |
| Verbindung Beispiel 1 | 0,50% |
| Perfume | 0,25% |
| Water | 55,75% |
| Polymer JR 400, Amerchol (INCI: Polyquaternium-10) | 0,20% |
| TEGO® Betain F 50, Evonik Goldschmidt GmbH, 38%-ig (INCI: Cocamidopropyl Betaine) | 8,00% |
| NaCl | 0,30% |
| Preservative | q.s. |

Formulierungsbeispiel 6) Clear Conditioning Shampoo

| | |
|---|---|
| TEXAPON® NSO, Cognis, 28%-ig (INCI: Sodium Laureth Sulfate) | 32,00% |
| VARISOFT® PATC, Evonik Goldschmidt GmbH (INCI: Palmitamidopropyltrimonium Chloride) | 1,50% |

(fortgesetzt)

| | |
|---|---|
| REWODERM® LI S 80, Evonik Goldschmidt GmbH (INCI: PEG-200 Hydrogenated Glyceryl Palmate; PEG-7 Glyceryl Cocoate) | 2,00% |
| Verbindung Beispiel 2 | 0,50% |
| Perfume | 0,25% |
| Water | 54,05% |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,20% |
| TEGO® Betain F 50, Evonik Goldschmidt GmbH, 38%-ig (INCI: Cocamidopropyl Betaine) | 8,00% |
| NaCl | 0,50% |
| Preservative | q.s. |

Formulierungsbeispiel 7) Clear Conditioning Shampoo

| | |
|---|---|
| TEXAPON® NSO, Cognis, 28%-ig (INCI: Sodium Laureth Sulfate) | 32,00% |
| REWODERM® LI S 80, Evonik Goldschmidt GmbH (INCI: PEG-200 Hydrogenated Glyceryl Palmate; PEG-7 Glyceryl Cocoate) | 2,00% |
| Verbindung Beispiel 2 | 0,50% |
| Perfume | 0,25% |
| Water | 55,55% |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,20% |
| TEGO® Betain F 50, Evonik Goldschmidt GmbH, 38%-ig (INCI: Cocamidopropyl Betaine) | 8,00% |
| NaCl | 0,50% |
| Preservative | q.s. |

Formulierungsbeispiel 8) Pearlized Shampoo

| | |
|---|---|
| TEXAPON® NSO, Cognis, 28%-ig (INCI: Sodium Laureth Sulfate) | 32,00% |
| Verbindung Beispiel 1 | 0,50% |
| Perfume | 0,25% |
| Water | 55,25% |
| TEGO® Betain F 50, Evonik Goldschmidt GmbH, 38%-ig (INCI: Cocamidopropyl Betaine) | 8, 00% |
| TEGO® Pearl N 300 Evonik Goldschmidt GmbH (INCI: Glycol Distearate; Laureth-4; Cocamidopropyl Betaine) | 2,00% |
| ANTIL® 171 Evonik Goldschmidt GmbH (INCI: PEG-18 Glyceryl Oleate/Cocoate) | 1,50% |
| NaCl | 0,50% |
| Preservative | q.s. |

Formulierungsbeispiel 9) 2 in 1 Shampoo

| | |
|---|---|
| TEXAPON® NSO, Cognis, 28%-ig (INCI: Sodium Laureth Sulfate) | 32,00% |
| VARISOFT® PATC, Evonik Goldschmidt GmbH (INCI: Palmitamidopropyltrimonium Chloride) | 1,50% |

(fortgesetzt)

| | |
|---|---|
| REWODERM® LI S 80, Evonik Goldschmidt GmbH (INCI: PEG-200 Hydrogenated Glyceryl Palmate; PEG-7 Glyceryl Cocoate) | 2,00% |
| Verbindung Beispiel 1 | 0,50% |
| Perfume | 0,25% |
| Water | 54,05% |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,20% |
| TEGO® Betain F 50, Evonik Goldschmidt GmbH, 38%-ig (INCI: Cocamidopropyl Betaine) | 8,00% |
| NaCl | 0,50% |
| Preservative | q.s. |

Formulierungsbeispiel 10) Rinse-Off Conditioner

| | |
|---|---|
| Water | 90,50% |
| VARISOFT® BT 85, Evonik Goldschmidt GmbH (INCI: Behentrimonium Chloride) | 3,00% |
| Verbindung Beispiel 1 | 1,50% |
| TEGO® Alkanol 1618, Evonik Goldschmidt GmbH (INCI: Cetearyl Alcohol) | 5,00% |
| Preservative, Perfume | q.s. |

Formulierungsbeispiel 11) Rinse-Off Conditioner

| | |
|---|---|
| Water | 90,20% |
| VARISOFT® EQ 65, Evonik Goldschmidt GmbH (INCI: Distearyl Dimonium Chloride, Cetearyl Alcohol) | 2,00% |
| VARISOFT@ BT 85, Evonik Goldschmidt GmbH (INCI: Behentrimonium Chloride) | 2,00% |
| Verbindung Beispiel 1 | 0,80% |
| TEGO® Alkanol 1618, Evonik Goldschmidt GmbH (INCI: Cetearyl Alcohol) | 5,00% |
| Preservative, Perfume | q.s. |

Formulierungsbeispiel 12) Rinse-Off Conditioner

| | |
|---|---|
| Water | 89,20% |
| VARISOFT® EQ 65, Evonik Goldschmidt GmbH (INCI: Distearyl Dimonium Chloride, Cetearyl Alcohol) | 2,00% |
| VARISOFT® BT 85, Evonik Goldschmidt GmbH (INCI: Behentrimonium Chloride) | 2,00% |
| ABIL® Quat 3272, Evonik Goldschmidt GmbH (INCI: Quaternium-80) | 1,00% |
| Verbindung Beispiel 1 | 0,80% |
| TEGO® Alkanol 1618, Evonik Goldschmidt GmbH (INCI: Cetearyl Alcohol) | 5,00% |
| Preservative, Perfume | q.s. |

Formulierungsbeispiel 13) Rinse-Off Conditioner

| | |
|---|---|
| TEGINACID® C, Evonik Goldschmidt GmbH (INCI: Ceteareth-25) | 0,50% |
| TEGO® Alkanol 16, Evonik Goldschmidt GmbH (INCI: Cetyl Alcohol) | 2,00% |
| TEGO® Amid S 18, Evonik Goldschmidt GmbH (INCI: Stearamidopropyl Dimethylamine) | 1,00% |
| Verbindung Beispiel 1 | 1,50% |
| Propylene Glycol | 2,00% |
| Citric Acid Monohydrate | 0,30% |
| Water | 92,70% |
| Preservative, Perfume | q.s. |

Formulierungsbeispiel 14) Rinse-Off Conditioner

| | |
|---|---|
| TEGINACID® C, Evonik Goldschmidt GmbH (INCI: Ceteareth-25) | 0,50% |
| TEGO® Alkanol 16, Evonik Goldschmidt GmbH (INCI: Cetyl Alcohol) | 5,00% |
| TEGOSOFT® DEC, Evonik Goldschmidt GmbH (INCI: Diethylhexyl Carbonate) | 1,00% |
| Verbindung Beispiel 2 | 1,50% |
| Water | 89,20% |
| TEGO® Cosmo C 100 Evonik Goldschmidt GmbH (INCI: Creatine) | 0,50% |
| Propylene Glycol | 2,00% |
| Citric Acid Monohydrate | 0,30% |
| Preservative, Perfume | q.s. |

Formulierungsbeispiel 15) Leave-In Conditioner Spray

| | |
|---|---|
| Lactic Acid, 80% | 0,40% |
| Water | 95,30% |
| TEGO® Amid S 18, Evonik Goldschmidt GmbH (INCI: Stearamidopropyl Dimethylamine) | 1,20% |
| TEGIN® G 1100 Pellets, Evonik Goldschmidt GmbH (INCI: Glycol Distearate) | 0,60% |
| TEGO® Care PS, Evonik Goldschmidt GmbH (INCI: Methyl Glucose Sesquistearate) | 1,20% |
| TEGOSOFT® DEC, Evonik Goldschmidt GmbH (INCI: Diethylhexyl Carbonate) | 0,30% |
| Verbindung Beispiel 1 | 1,00% |
| Preservative, Perfume | q.s. |

Formulierungsbeispiel 16) Leave-In Conditioner Spray

| | |
|---|---|
| TAGAT® CH-40, Evonik Goldschmidt GmbH (INCI: PEG-40 Hydrogenated Castor Oil) | 2,00% |
| Ceramide VI, Evonik Goldschmidt GmbH (INCI: Ceramide 6 II) | 0,05% |
| Perfume | 0,20% |
| Water | 90,95% |
| Verbindung Beispiel 1 | 0,50% |
| LACTIL® Evonik Goldschmidt GmbH (INCI: Sodium Lactate; Sodium PCA; Glycine; Fructose; Urea; Niacinamide; Inositol; Sodium benzoate; Lactic Acid) | 2,00% |

(fortgesetzt)

| | |
|---|---|
| TEGO® Betain F 50 Evonik Goldschmidt GmbH 38% (INCI: Cocamidopropyl Betaine) | 2,30% |
| Citric Acid (10% in water) | 2,00% |

Formulierungsbeispiel 17) Leave-In Conditioner Foam

| | |
|---|---|
| Verbindung Beispiel 1 | 0,50% |
| TAGAT® CH-40, Evonik Goldschmidt GmbH (INCI: PEG-40 Hydrogenated Castor Oil) | 0,50% |
| Perfume | 0,30% |
| TEGO® Betain 810, Evonik Goldschmidt GmbH (INCI: Capryl/Capramidopropyl Betaine) | 2,00% |
| Water | 94,00% |
| TEGO® Cosmo C 100, Evonik Goldschmidt GmbH (INCI: Creatine) | 0,50% |
| TEGOCEL® HPM 50, Evonik Goldschmidt GmbH (INCI: Hydroxypropyl Methylcellulose) | 0,30% |
| VARISOFT® 300, Evonik Goldschmidt GmbH (INCI: Cetrimonium Chloride) | 1,30% |
| LACTIL® Evonik Goldschmidt GmbH (INCI: Sodium Lactate; Sodium PCA; Glycine; Fructose; Urea; Niacinamide; Inositol; Sodium benzoate; Lactic Acid) | 0, 50% |
| Citric Acid (30% in water) | 0,10% |
| Preservative | q.s. |

Formulierungsbeispiel 18) Strong Hold Styling Gel

| | |
|---|---|
| TEGO® Carbomer 141, Evonik Goldschmidt GmbH (INCI: Carbomer) | 1,20% |
| Water | 67,00% |
| NaOH, 25% | 2,70% |
| PVP/VA W-735, ISP (INCI: PVP/VA Copolymer) | 16,00% |
| Verbindung Beispiel 1 | 0,50% |
| Alcohol Denat. | 10,00% |
| TAGAT® O 2 V, Evonik Goldschmidt GmbH (INCI: PEG-20 Glyceryl Oleate) | 2,00% |
| Perfume | 0,30% |
| ABIL® B 88183, Evonik Goldschmidt GmbH (INCI: PEG/PPG-20/6 Dimethicone) | 0,30% |
| Preservative | q.s. |

Formulierungsbeispiel 19) Schaumiges Körperpflegemittel

| | |
|---|---|
| TEXAPON® NSO, Cognis, 28%-ig (INCI: Sodium Laureth Sulfate) | 14,30% |
| Perfume | 0,30% |
| Verbindung Beispiel 1 | 0,50% |
| REWOTERIC® AM C, Evonik Goldschmidt GmbH, 32%-ig (INCI: Sodium Cocoamphoacetate) | 8,00% |
| Water | 74,90% |
| TEGOCEL® HPM 50, Evonik Goldschmidt GmbH (INCI: Hydroxypropyl Methylcellulose) | 0,50% |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 1,00% |

(fortgesetzt)

| | |
|---|---|
| Citric Acid Monohydrate | 0,50% |

Formulierungsbeispiel 20) Körperpflegemittel

| | |
|---|---|
| TEXAPON® NSO, Cognis, 28%-ig (INCI: Sodium Laureth Sulfate) | 30,00% |
| TEGOSOFT® PC 31, Evonik Goldschmidt GmbH (INCI: Polyglyceryl-3 Caprate) | 0,50% |
| Verbindung Beispiel 2 | 0,50% |
| Perfume | 0,30% |
| Water | 53,90% |
| TEGOCEL® HPM 4000, Evonik Goldschmidt GmbH (INCI: Hydroxypropyl Methylcellulose) | 0,30% |
| REWOTERIC® AM C, Evonik Goldschmidt GmbH, 32%-ig (INCI: Sodium Cocoamphoacetate) | 10,00% |
| Citric Acid Monohydrate | 0,50% |
| REWODERM® LI S 80, Evonik Goldschmidt GmbH (INCI: PEG-200 Hydrogenated Glyceryl Palmate; PEG-7 Glyceryl Cocoate) | 2,00% |
| TEGO® Pearl N 300, Evonik Goldschmidt GmbH (INCI: Glycol Distearate; Laureth-4; Cocamidopropyl Betaine) | 2,00% |

Formulierungsbeispiel 21) Schaumiges Körperpflegemittel

| | |
|---|---|
| TEXAPON® NSO, Cognis, 28%-ig (INCI: Sodium Laureth Sulfate) | 14,30% |
| Perfume | 0,30% |
| Verbindung Beispiel 1 | 0,50% |
| REWOTERIC® AM C, Evonik Goldschmidt GmbH, 32%-ig (INCI: Sodium Cocoamphoacetate) | 8,00% |
| Water | 75,10% |
| Polyquaternium-7 | 0,30% |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 1,00% |
| Citric Acid Monohydrate | 0,50% |

Formulierungsbeispiel 22) Mild Foam Bath

| | |
|---|---|
| TEXAPON® NSO, Cognis, 28%-ig (INCI: Sodium Laureth Sulfate) | 27,00% |
| REWOPOL® SB FA 30, Evonik Goldschmidt GmbH, 40%-ig (INCI: Disodium Laureth Sulfosuccinate) | 12,00% |
| TEGOSOFT® LSE 65 K SOFT, Evonik Goldschmidt GmbH (INCI: Sucrose Cocoate) | 2,00% |
| Water | 39,00% |
| REWOTERIC® AM C, Evonik Goldschmidt GmbH, 32%-ig (INCI: Sodium Cocoamphoacetate) | 13,00% |
| Verbindung Beispiel 1 | 0,50% |
| Citric Acid (30% in water) | 3,00% |
| ANTIL® 171 Evonik Goldschmidt GmbH (INCI: PEG-18 Glyceryl Oleate/Cocoate) | 1,50% |

(fortgesetzt)

| | |
|---|---|
| TEGO® Pearl N 300 Evonik Goldschmidt GmbH (INCI: Glycol Distearate; Laureth-4; Cocamidopropyl Betaine) | 2,00% |

Formulierungsbeispiel 23) Schaumiges Körperpflegemittel

| | |
|---|---|
| TEGOCEL® HPM 50, Evonik Goldschmidt GmbH (INCI: Hydroxypropyl Methylcellulose) | 0,50% |
| Water | 80,10% |
| Perfume | 0,20% |
| Verbindung Beispiel 1 | 0,50% |
| TEGOSOFT® GC, Evonik Goldschmidt GmbH, (INCI: PEG-7 Glyceryl Cocoate) | 1,30% |
| TEGO® Betain 810, Evonik Goldschmidt GmbH (INCI: Capryl/Capramidopropyl Betaine) | 16,90% |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 0,50% |
| Preservative | q.s. |

Formulierungsbeispiel 24) Clear Shampoo

| | |
|---|---|
| TEXAPON® NSO, Cognis, 28%-ig (INCI: Sodium Laureth Sulfate) | 32,00% |
| Verbindung Beispiel 2 | 0,50% |
| Perfume | 0,25% |
| Water | 56,05% |
| TEGO® Betain F 50, Evonik Goldschmidt GmbH, 38%-ig (INCI: Cocamidopropyl Betaine) | 8,00% |
| ANTIL® 171 Evonik Goldschmidt GmbH (INCI: PEG-18 Glyceryl Oleate/Cocoate) | 2,50% |
| NaCl | 0,70% |
| Preservative | q.s. |

Formulierungsbeispiel 25) Clear Shampoo

| | |
|---|---|
| TEXAPON® NSO, Cognis, 28%-ig (INCI: Sodium Laureth Sulfate) | 32,00% |
| Verbindung Beispiel 1 | 0,50% |
| Perfume | 0,25% |
| Water | 55,35% |
| REWOTERIC® AMC, Evonik Goldschmidt GmbH, (INCI: Sodium Cocoamphoacetate) | 9,40% |
| ANTIL® 171 Evonik Goldschmidt GmbH (INCI: PEG-18 Glyceryl Oleate/Cocoate) | 2,50% |
| Preservative | q.s. |

Formulierungsbeispiel 25) Clear Shampoo

| | |
|---|---|
| TEXAPON® NSO, Cognis, 28%-ig (INCI: Sodium Laureth Sulfate) | 17,90% |
| Verbindung Beispiel 1 | 0,50% |
| Perfume | 0,25% |
| Water | 62,50% |

(fortgesetzt)

| | |
|---|---|
| TEGO® Betain F 50, Evonik Goldschmidt GmbH, 38%-ig (INCI: Cocamidopropyl Betaine) | 6,60% |
| REWOPOL® SB FA 30, Evonik Goldschmidt GmbH, (INCI: Disodium Laureth Sulfosuccinate) | 6,25% |
| ANTIL® 171 Evonik Goldschmidt GmbH (INCI: PEG-18 Glyceryl Oleate/Cocoate) | 5,00% |
| NaCl | 1,00% |
| Preservative | q.s. |

Formulierungsbeispiel 26) Rinse-Off Conditioner

| | |
|---|---|
| Water | 89,20% |
| VARISOFT® EQ 65, Evonik Goldschmidt GmbH (INCI: Distearyl Dimonium Chloride, Cetearyl Alcohol) | 2,00% |
| VARISOFT® BT 85, Evonik Goldschmidt GmbH (INCI: Behentrimonium Chloride) | 2,00% |
| ABIL® OSW 5, Evonik Goldschmidt GmbH (INCI: Cyclopentasiloxane; Dimethiconol) | 1,00% |
| Verbindung Beispiel 1 | 0,80% |
| TEGO® Alkanol 1618, Evonik Goldschmidt GmbH (INCI: Cetearyl Alcohol) | 5,00% |
| Preservative, Perfume | q.s. |

Formulierungsbeispiel 27) Rinse-Off Conditioner

| | |
|---|---|
| Water | 89,20% |
| VARISOFT® EQ 65, Evonik Goldschmidt GmbH (INCI: Distearyl Dimonium Chloride, Cetearyl Alcohol) | 2,00% |
| VARISOFT® BT 85, Evonik Goldschmidt GmbH (INCI: Behentrimonium Chloride) | 2,00% |
| ABIL® Soft AF 100, Evonik Goldschmidt GmbH (INCI: Methoxy PEG/PPG-7/3 Aminopropyl Dimethicone) | 1,00% |
| Verbindung Beispiel 1 | 0,80% |
| TEGO® Alkanol 1618, Evonik Goldschmidt GmbH (INCI: Cetearyl Alcohol) | 5,00% |
| Preservative, Perfume | q.s. |

Formulierungsbeispiel 28) Rinse-Off Conditioner

| | |
|---|---|
| Water | 89,20% |
| VARISOFT® EQ 65, Evonik Goldschmidt GmbH (INCI: Distearyl Dimonium Chloride, Cetearyl Alcohol) | 2,00% |
| VARISOFT® BT 85, Evonik Goldschmidt GmbH (INCI: Behentrimonium Chloride) | 2,00% |
| SF 1708, Momentive (INCI: Amodimethicone) | 1,00% |
| Verbindung Beispiel 1 | 0,80% |
| TEGO® Alkanol 1618, Evonik Goldschmidt GmbH (INCI: Cetearyl Alcohol) | 5,00% |
| Preservative, Perfume | q.s. |

Formulierungsbeispiel 29) Conditioning Shampoo

| | |
|---|---|
| TEXAPON® NSO, Cognis, 28%-ig (INCI: Sodium Laureth Sulfate) | 27,00% |
| Plantacare 818 UP, Cognis 51.4%-ig (INCI: Coco Glucoside) | 5,00% |
| Verbindung Beispiel 2 | 1,50% |
| Perfume | 0,25% |
| Water | 56,55% |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,20% |
| TEGO® Betain F 50, Evonik Goldschmidt GmbH, 38%-ig (INCI: Cocamidopropyl Betaine) | 8,00% |
| NaCl | 0,50% |
| Preservative | q.s. |

Formulierungsbeispiel 30) Conditioning Shampoo

| | |
|---|---|
| Plantacare 818 UP, Cognis 51.4%-ig (INCI: Coco Glucoside) | 18,00% |
| Verbindung Beispiel 2 | 1,50% |
| Perfume | 0,25% |
| Water | 70,55% |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,20% |
| TEGO® Betain F 50, Evonik Goldschmidt GmbH, 38%-ig (INCI: Cocamidopropyl Betaine) | 8,00% |
| NaCl | 0,50% |
| Preservative | q.s. |

## Patentansprüche

**1.** Mindestens eine quatäre Ammoniumgruppe enthaltendes Polysiloxan der allgemeinen Formel (I)

$$M_a\, M'_{a1}\, M''_{a2}\, M'''_{a3}\, D_b\, D'_{b1}\, D''_{b2}\, D'''_{b3}\, T_c\, T'_{c1}\, Q_d \qquad \text{Formel (I),}$$

wobei

$M = (R^1_3\ Si\ O_{1/2})$
$M' = (R^2R^1_2\ Si\ O_{1/2})$
$M'' = (R^3R^1_2\ Si\ O_{1/2})$
$M''' = (R^4R^1_2\ Si\ O_{1/2})$
$D = (R^1_2\ Si\ O_{2/2})$
$D' = (R^2R^1\ Si\ O_{2/2})$
$D'' = (R^3R^1\ Si\ O_{2/2})$
$D''' = (R^4R^1\ Si\ O_{2/2})$
$T = (R^5\ Si\ O_{3/2})$
$T' = (R^2\ Si\ O_{3/2})$
$Q = (Si\ O_{4/2})$
a = 0 bis 32; bevorzugt 0 bis 22, insbesondere 0 bis 12;
a1 = 0 bis 10, bevorzugt 0 bis 5, insbesondere 0;
a2 = 0 bis 32; bevorzugt 0 bis 22, insbesondere 1 bis 12;
a3 = 0 bis 10; bevorzugt 0 bis 5, insbesondere 0; mit der Maßgabe, dass

$$a + a1 + a2 + a3 > 3, \text{ bevorzugt } > 4;$$

b = 1 bis 600, bevorzugt 10 bis 500, insbesondere 20 bis 400;
b1 = 0 bis 10, bevorzugt 0 bis 5, insbesondere 0;
b2 = 0 bis 80, bevorzugt 0 bis 50, insbesondere 0 bis 10;
b3 = 0 bis 20, bevorzugt 0 bis 10, insbesondere 0;
c = 0 bis 30, bevorzugt 1 bis 20, insbesondere 2 bis 15;
c1 = 0 bis 10, bevorzugt 0 bis 5, insbesondere 0;
d = 0 bis 15, bevorzugt 1 bis 12, insbesondere 2 bis 10;

mit der Maßgabe, dass

$$a2 + b2 > 3$$

und

$$c + c1 + d > 1, \text{ bevorzugt } > 2, \text{ insbesondere } \geq 3;$$

$R^1$ = unabhängig voneinander gleiche oder verschiedene lineare oder verzweigte, gegebenenfalls aromatische Kohlenwasserstoffreste mit 1 bis 30 Kohlenstoffatomen, bevorzugt Methyl oder Phenyl, insbesondere Methyl;
$R^2$ = unabgängig voneinander gleiche oder verschiedene Alkoxy- oder Acyloxyreste, wie beispielsweise Methoxy-, Ethoxy-, n-Propoxy oder iso-Propoxyreste, Acetoxy, insbesondere Ethoxy oder iso-Propoxyreste;
$R^3$ = unabhängig voneinander gleiche oder verschiedene organische Reste, die quatäre Ammoniumfunktionen tragen;
$R^4$ = unabhängig voneinander gleiche oder verschiedene organische Epoxy-Reste;
$R^5$ = unabhängig voneinander gleiche oder verschiedene Reste $R^1$, $R^3$ oder $R^4$, bevorzugt $R^1$, insbesondere Methyl, Phenyl, Dodecyl oder Hexadecyl

sind.

**2.** Polysiloxan gemäß Anspruch 2, **dadurch gekennzeichnet, dass** $R^4$ gleiche oder verschiedene Reste ausgewählt aus der Gruppe

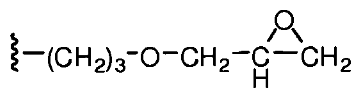

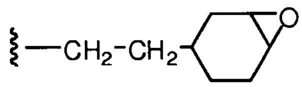

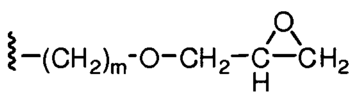

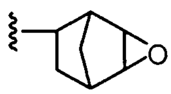

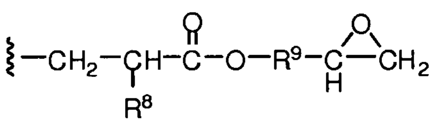

.

**3.** Polysiloxan gemäß Anspruch 2 oder 3, **dadurch gekennzeichnet, dass** $R^3$ Gruppen mit dem Aufbau

- $R^6$-$R^7$ sind, worin

$R^6$ vorzugsweise gleiche oder verschiedene zweiwertige Reste sind, ausgewählt aus der Gruppe

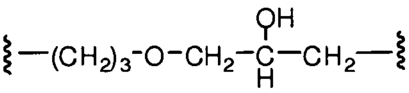
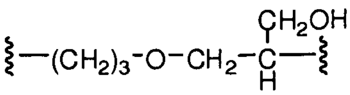

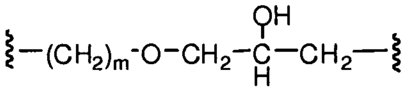
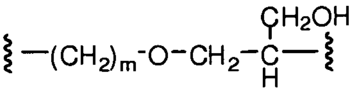

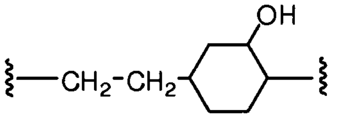
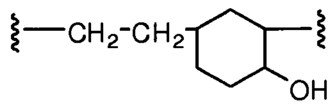

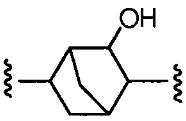
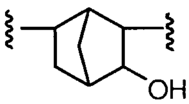

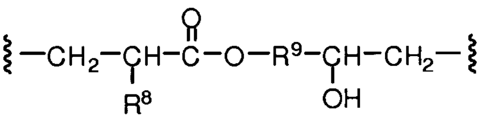
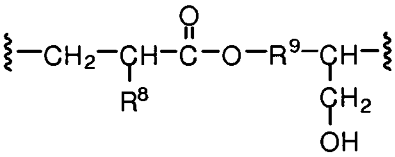
;

$R^7$ ausgewählt ist aus der Gruppe bestehend aus

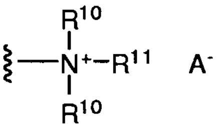
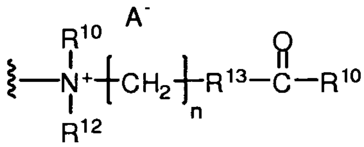

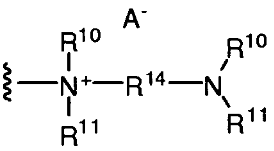
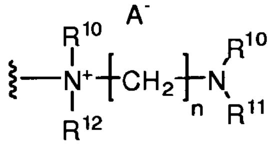
;

$R^8$ sind gleiche oder verschiedene Reste aus der Gruppe Wasserstoff oder Alkyl mit 1 bis 6 C-Atomen, bevorzugt Methyl;

$R^9$ sind gleiche oder verschiedene zweiwertige Kohlenwasserstoffreste, die gegebenenfalls Etherfunktionen enthalten, bevorzugt Methylen;

$R^{10}$, $R^{11}$, $R^{12}$ sind jeweils unabhängig voneinander Wasserstoff oder Alkylreste mit 1 bis 30 C-Atomen oder Reste der Formel

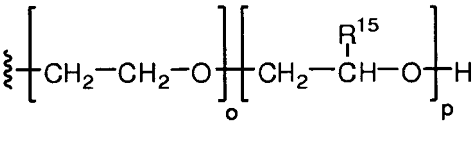
;

$R^{13}$ sind gleiche oder verschiedene Reste aus der Gruppe -O-; -$NR^{16}$-;

$R^{14}$ sind gleiche oder verschiedene gegebenenfalls verzweigte divalente Kohlenwasserstoffreste, bevorzugt Ethylen oder Propylen;

$R^{15}$ sind gleiche oder verschiedene Alkyl-, Aryl- oder Alkarylreste mit 1 bis 30 C-Atomen, die gegebenenfalls Etherfunktionen enthalten, bevorzugt Methyl, Ethyl oder Phenyl, insbesondere Methyl;
$R^{16}$ sind gleiche oder verschiedene Reste aus der Gruppe Wasserstoff oder Alkyl mit 1 bis 6 C-Atomen;
m = 2 bis 18;
n = 2 bis 18, bevorzugt 3;
o = 0 bis 30, bevorzugt 0 bis 10, insbesondere 1 bis 3;
p = 0 bis 30, bevorzugt 0 bis 10;
$A^-$ sind gleiche oder verschiedene Gegenionen zu den positiven Ladungen an den quaternierten Stickstoffgruppen, ausgewählt aus anorganischen oder organischen Anionen der Säuren HA, sowie deren Derivate.

**4.** Verfahren zur Herstellung eines Polysiloxans gemäß mindestens einem der vorherigen Ansprüche umfassend die Verfahrensschritte

A) Herstellung eines SiH-Gruppen-haltigen, über mindestens zwei Einheiten, ausgewählt aus T- und Q-Einheiten, verzweigten Siloxangrundgerüstes durch Equilibrierung und Kondensation einer Mischung, enthaltend die Komponenten

a) mindestens ein SiH-funktionelles Siloxan,
b) mindestens ein SiH-Funktion-freies Siloxan, und entweder Komponente c) oder Komponente d), oder beide Komponenten c) und d), wobei
c) mindestens ein Tetraalkoxysilan,
d) mindestens ein Trialkoxysilan unter Zugabe von Wasser und mindestens einem geeigneten Katalysators.

B) Hydrosilylierung der SiH-funktionellen Siloxane aus Verfahrensschritt A) mit mindestens einem ungesättigten Epoxid,
C) Quaternisierung der Epoxysiloxane aus Verfahrensschritt B) mit mindestens einem tertiären Amin.

**5.** Verwendung mindestens einer Verbindung gemäß mindestens einem der Ansprüche 1 bis 3 oder mindestens eines Polysiloxans erhältlich nach einem Verfahren gemäß Anspruch 4 zur Herstellung von kosmetischen, pharmazeutischen oder dermatologischen Kompositionen.

**6.** Verwendung mindestens einer Verbindung gemäß mindestens einem der Ansprüche 1 bis 3 oder mindestens eines Polysiloxans erhältlich nach einem Verfahren gemäß Anspruch 4 als Pflegewirkstoff in Pflege- und Reinigungsformulierungen.

**7.** Verwendung mindestens einer Verbindung gemäß mindestens einem der Ansprüche 1 bis 3 oder mindestens eines Polysiloxans erhältlich nach einem Verfahren gemäß Anspruch 4 als Konditioniermittel für Haarbehandlungsmittel und Haarnachbehandlungsmittel sowie als Mittel zur Verbesserung der Haarstruktur.

**8.** Kosmetische, pharmazeutische oder dermatologische Komposition, insbesondere tensidhaltige wässrige Pflege- und Reinigungsformulierungen, Haarbehandlungsmittel und Haarnachbehandlungsmittel zum Ausspülen oder zum Verbleib im Haar, enthaltend mindestens eine Verbindung gemäß mindestens einem der Ansprüche 1 bis 3 oder mindestens ein Polysiloxan erhältlich nach einem Verfahren gemäß Anspruch 4.

**9.** Reinigungs- und Pflegeformulierungen für Haushalt, industrielle und institutionelle Anwendungen, insbesondere Weichspülmittel, enthaltend mindestens eine Verbindung gemäß mindestens einem der Ansprüche 1 bis 3 oder mindestens ein Polysiloxan erhältlich nach einem Verfahren gemäß Anspruch 4.

## Claims

**1.** Polysiloxane comprising at least one quaternary ammonium group and of the general formula (I)

$$M_a\, M'_{a1}\, M''_{a2}\, M'''_{a3}\, D_b\, D'_{b1}\, D''_{b2}\, D'''_{b3}\, T_c\, T'_{c1}\, Q_d \qquad \text{formula (I)},$$

where

M = $(R^1_3\ Si\ O_{1/2})$

M' = ($R^2R^1_2$ Si $O_{1/2}$)
M" = ($R^3R^1_2$ Si $O_{1/2}$)
M"' = ($R^4R^1_2$ Si $O_{1/2}$)
D = ($R^1_2$ Si $O_{2/2}$)
D' = ($R^2R^1$ Si $O_{2/2}$)
D" = ($R^3R^1$ Si $O_{2/2}$)
D"' = ($R^4R^1$ Si $O_{2/2}$)
T = ($R^5$ Si $O_{3/2}$)
T' = ($R^2$ Si $O_{3/2}$)
Q = (Si $O_{4/2}$)
a = 0 to 32; preferably 0 to 22, more particularly 0 to 12;
a1 = 0 to 10, preferably 0 to 5, more particularly 0;
a2 = 0 to 32; preferably 0 to 22, more particularly 1 to 12;
a3 = 0 to 10; preferably 0 to 5, more particularly 0;

with the proviso that

$$a + a1 + a2 + a3 > 3, \text{ preferably } > 4;$$

b = 1 to 600, preferably 10 to 500, more particularly 20 to 400;
b1 = 0 to 10, preferably 0 to 5, more particularly 0;
b2 = 0 to 80, preferably 0 to 50, more particularly 0 to 10;
b3 = 0 to 20, preferably 0 to 10, more particularly 0;
c = 0 to 30, preferably 1 to 20, more particularly 2 to 15;
c1 = 0 to 10, preferably 0 to 5, more particularly 0;
d = 0 to 15, preferably 1 to 12, more particularly 2 to 10;

with the proviso that

$$a2 + b2 > 3$$

and

$$c + c1 + d > 1, \text{ preferably } > 2, \text{ more particularly } \geq 3;$$

$R^1$ = independently of one another identical or different linear or branched, optionally aromatic hydrocarbon radicals having 1 to 30 carbon atoms, preferably methyl or phenyl, more particularly methyl;
$R^2$ = independently of one another identical or different alkoxy or acyloxy radicals, such as, for example, methoxy, ethoxy, n-propoxy or isopropoxy radicals, acetoxy, more particularly ethoxy or isopropoxy radicals;
$R^3$ = independently of one another identical or different organic radicals which carry quaternary ammonium functions;
$R^4$ = independently of one another identical or different organic epoxy radicals;
$R^5$ = independently of one another identical or different radicals $R^1$, $R^3$ or $R^4$, preferably $R^1$, more particularly methyl, phenyl, dodecyl or hexadecyl.

2. Polysiloxane according to Claim 2, **characterized in that** $R^4$ radicals are identical or different radicals selected from the group

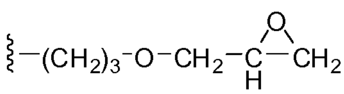

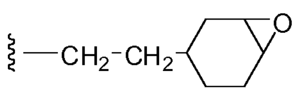

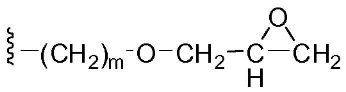

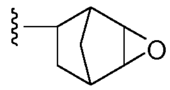

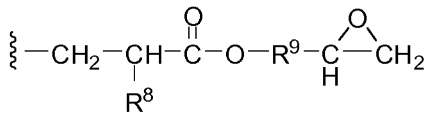

**3.** Polysiloxane according to Claim 2 or 3, **characterized in that** $R^3$ groups are groups with the structure $-R^6-R^7$, in which

$R^6$ radicals are preferably identical or different divalent radicals selected from the group

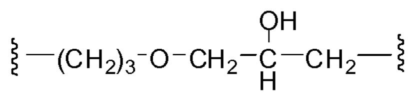

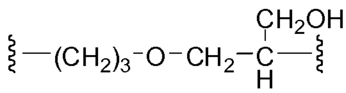

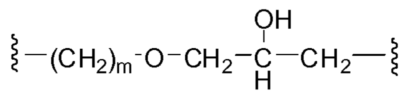

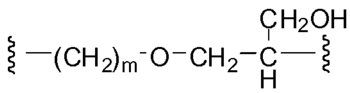

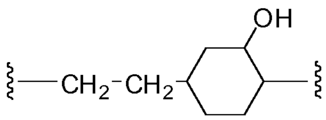

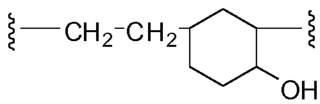

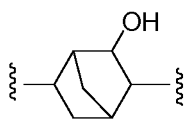

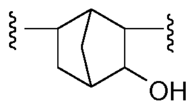

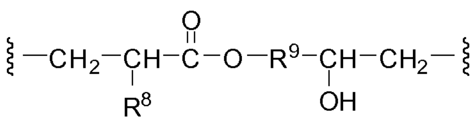

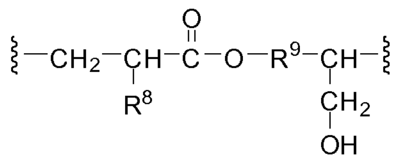

;

$R^7$ is selected from the group consisting of

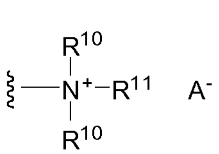

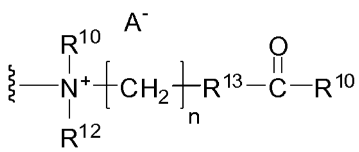

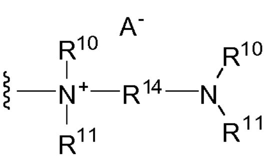

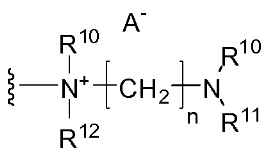

;

$R^8$ are identical or different radicals from the group of hydrogen or alkyl having 1 to 6 C atoms, preferably methyl; $R^9$ are identical or different divalent hydrocarbon radicals which optionally contain ether functions, preferably

methylene;
$R^{10}$, $R^{11}$, and $R^{12}$ are in each case independently of one another hydrogen or alkyl radicals having 1 to 30 C atoms, or radicals of the formula

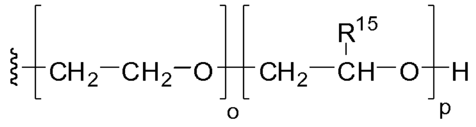
;

$R^{13}$ are identical or different radicals from the group -O-; $-NR^{16}-$;
$R^{14}$ are identical or different optionally branched divalent hydrocarbon radicals, preferably ethylene or propylene;
$R^{15}$ are identical or different alkyl, aryl or alkaryl radicals having 1 to 30 C atoms, which optionally contain ether functions, preferably methyl, ethyl or phenyl, more particularly methyl;
$R^{16}$ are identical or different radicals from the group of hydrogen or alkyl having 1 to 6 C atoms;
m = 2 to 18;
n = 2 to 18, preferably 3;
o = 0 to 30, preferably 0 to 10, more particularly 1 to 3;
p = 0 to 30, preferably 0 to 10;
$A^-$ are identical or different counterions to the positive charges on the quaternized nitrogen groups, selected from inorganic or organic anions of the acids HA, and also derivatives thereof.

**4.** Process for preparing a polysiloxane according to at least one of the preceding claims, comprising the steps of

A) preparing an SiH-group-containing siloxane framework, branched via at least two units selected from T and Q units, by equilibration and condensation of a mixture comprising the components

a) at least one SiH-functional siloxane,
b) at least one SiH-function-free siloxane, and either component c) or component d), or both components c) and d), where
c) is at least one tetraalkoxysilane,
d) is at least one trialkoxysilane with addition of water and at least one suitable catalyst,

B) hydrosilylating the SiH-functional siloxanes from process step A) with at least one unsaturated epoxide,
C) quaternizing the epoxysiloxanes from process step B) with at least one tertiary amine.

**5.** Use of at least one compound according to at least one of Claims 1 to 3 or of at least one polysiloxane obtainable by a process according to Claim 4 for producing cosmetic, pharmaceutical or dermatological compositions.

**6.** Use of at least one compound according to at least one of Claims 1 to 3 or of at least one polysiloxane obtainable by a process according to Claim 4 as an active care ingredient in care and cleaning formulations.

**7.** Use of at least one compound according to at least one of Claims 1 to 3 or of at least one polysiloxane obtainable by a process according to Claim 4 as conditioning agents for hair treatment compositions and hair aftertreatment compositions and also as agents for improving hair structure.

**8.** Cosmetic, pharmaceutical or dermatological composition, more particularly surfactant-containing aqueous care and cleaning formulations, hair treatment compositions and hair aftertreatment compositions to be rinsed out of or left in the hair, comprising at least one compound according to at least one of Claims 1 to 3 or at least one polysiloxane obtainable by a process according to Claim 4.

**9.** Cleansing and care formulations for household, industrial and institutional applications, more particularly fabric softeners, comprising at least one compound according to at least one of Claims 1 to 3 or at least one polysiloxane obtainable by a process according to Claim 4.

## Revendications

**1.** Polysiloxane contenant au moins un groupe d'ammonium quaternaire de formule générale (I)

$$\mathbf{M_a\ M''_{a1}\ M''_{a2}\ M'''_{a3}\ D_b\ D'_{b1}\ D''_{b2}\ D'''_{b3}\ T_c\ T'_{c1}\ Q_d} \qquad \text{Formule (I)}$$

dans laquelle

M = $(R^1{}_3SiO_{1/2})$
M' = $(R^2R^1{}_2SiO_{1/2})$
M" = $(R^3R^1{}_2SiO_{1/2})$
M"' = $(R^4R^1{}_2SiO_{1/2})$
D = $(R^1{}_2SiO_{2/2})$
D' = $(R^2R^1SiO_{2/2})$
D" = $(R^3R^1SiO_{2/2})$
D"' = $(R^4R^1SiO_{2/2})$
T = $(R^5SiO_{3/2})$
T' = $(R^2SiO_{3/2})$
Q = $(SiO_{4/2})$
a = 0 à 32 ; de préférence 0 à 22, en particulier 0 à 12 ;
a1 = 0 à 10, de préférence 0 à 5, en particulier 0 ;
a2 = 0 à 32 ; de préférence 0 à 22, en particulier 1 à 12 ;
a3 = 0 à 10 ; de préférence 0 à 5, en particulier 0 ;

à condition que a + a1 + a2 + a3 > 3, de préférence > 4 ;

b = 1 à 600 ; de préférence 10 à 500, en particulier 20 à 400 ;
b1 = 0 à 10, de préférence 0 à 5, en particulier 0 ;
b2 = 0 à 80 ; de préférence 0 à 50, en particulier 0 à 10 ;
b3 = 0 à 20, de préférence 0 à 10, en particulier 0 ;
c = 0 à 30 ; de préférence 1 à 20, en particulier 2 à 15 ;
c1 = 0 à 10, de préférence 0 à 5, en particulier 0 ;
d = 0 à 15 ; de préférence 1 à 12, en particulier 2 à 10 ;

à condition que a2 + b2 > 3 et c + c1 + d > 1, de préférence > 2, en particulier ≥ 3 ;

$R^1$ = indépendamment l'un de l'autre, des radicaux hydrocarbonés identiques ou différents, linéaires ou ramifiés, le cas échéant aromatiques, comprenant 1 à 30 atomes de carbone, de préférence méthyle ou phényle, en particulier méthyle ;
$R^2$ = indépendamment l'un de l'autre, des radicaux alcoxy ou acyloxy identiques ou différents, tels que par exemple des radicaux méthoxy, éthoxy, n-propoxy ou iso-propoxy, acétoxy, en particulier des radicaux éthoxy ou iso-propoxy ;
$R^3$ = indépendamment l'un de l'autre, des radicaux organiques identiques ou différents, qui portent des fonctions d'ammonium quaternaire ;
$R^4$ = indépendamment l'un de l'autre, des radicaux époxy organiques identiques ou différents :
$R^5$ = indépendamment l'un de l'autre, des radicaux $R^1$, $R^3$ ou $R^4$, de préférence $R^1$, en particulier méthyle, phényle, dodécyle ou hexadécyle.

**2.** Polysiloxane selon la revendication 2, **caractérisé en ce que** $R^4$ représente des radicaux identiques ou différents, choisis dans le groupe

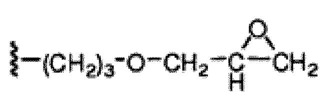

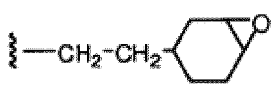

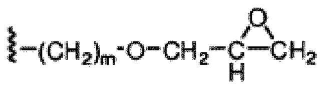

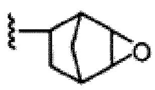

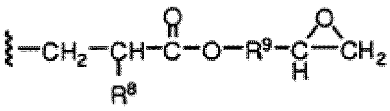

.

**3.** Polysiloxane selon la revendication 2 ou 3, **caractérisé en ce que** les groupes $R^3$ présentent la structure $-R^6-R^7$ dans laquelle

$R^6$ représentent de préférence des radicaux divalents, identiques ou différents, choisis dans le groupe

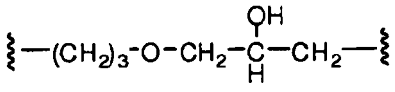

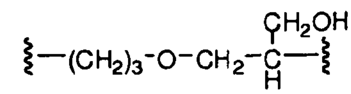

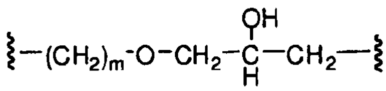

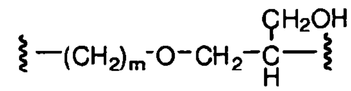

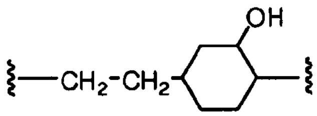

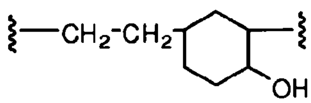

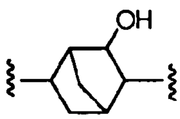

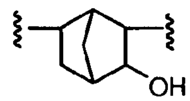

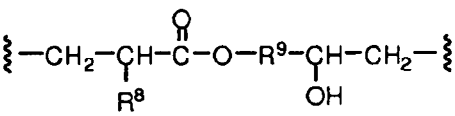

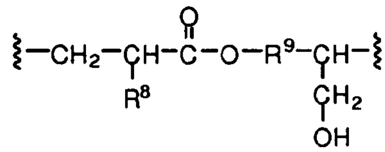

;

$R^7$ est choisi dans le groupe constitué par :

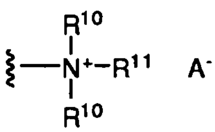

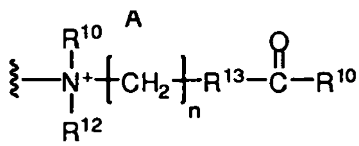

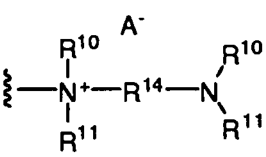

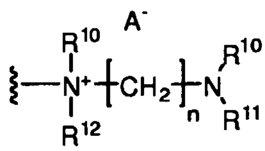

;

$R^8$ représentent des radicaux identiques ou différents du groupe formé par hydrogène ou alkyle comprenant 1 à 6 atomes de carbone, de préférence méthyle ;
$R^9$ représentent des radicaux hydrocarbonés divalents, identiques ou différents, qui contiennent le cas échéant

des fonctions éther, de préférence méthylène ;

$R^{10}$, $R^{11}$, $R^{12}$ représentent, à chaque fois indépendamment les uns des autres, hydrogène ou des radicaux alkyle comprenant 1 à 30 atomes de carbone ou des radicaux de formule

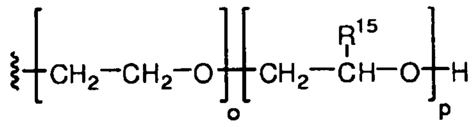

$R^{13}$ représentent des radicaux identiques ou différents du groupe formé par -O- ; $-NR^{16}-$ ;

$R^{14}$ représentent des radicaux hydrocarbonés divalents, identiques ou différents, le cas échéant ramifiés, de préférence éthylène ;

$R^{15}$ représentent des radicaux alkyle, aryle ou alkaryle identiques ou différents comprenant 1 à 30 atomes de carbone, qui contiennent le cas échéant des fonctions éther, de préférence méthyle, éthyle ou phényle, en particulier méthyle ;

$R^{16}$ représentent des radicaux identiques ou différents du groupe formé par hydrogène ou alkyle comprenant 1 à 6 atomes de carbone ;

m = 2 à 18 ;

n = 2 à 18, de préférence 3 ;

o = 0 à 30 ; de préférence 0 à 10, en particulier 1 à 3 ;

p = 0 à 30, de préférence 0 à 10 ;

$A^-$ représentent des contre-ions, identiques ou différents, des charges positives sur les groupes d'azote quaternisés, choisis parmi les anions inorganiques ou organiques des acides HA, ainsi que leurs dérivés.

**4.** Procédé pour la préparation d'un polysiloxane selon au moins l'une quelconque des revendications précédentes, comprenant les étapes de procédé consistant à :

A) préparer une structure de base siloxane, contenant des groupes SiH, ramifiée via au moins deux unités, choisies parmi les unités T et Q, par équilibrage et condensation d'un mélange, contenant les composants

a) au moins un siloxane à fonctionnalité SiH,

b) au moins un siloxane exempt de fonction SiH et, soit le composant c), soit le composant d), soit les deux composants c) et d), où

c) représente au moins un tétraalcoxysilane,

d) représente au moins un trialcoxysilane, avec addition d'eau et d'au moins un catalyseur approprié,

B) hydrosilyler les siloxanes à fonctionnalité SiH de l'étape de procédé A) avec au moins un époxyde insaturé,

C) quaterniser les époxysiloxanes de l'étape de procédé B) avec au moins une amine tertiaire.

**5.** Utilisation d'au moins un composé selon au moins l'une quelconque des revendications 1 à 3 ou d'au moins un polysiloxane pouvant être obtenu selon un procédé selon la revendication 4 pour la préparation de compositions cosmétiques, pharmaceutiques ou dermatologiques.

**6.** Utilisation d'au moins un composé selon au moins l'une quelconque des revendications 1 à 3 ou d'au moins un polysiloxane pouvant être obtenu selon un procédé selon la revendication 4 comme substance active de soin dans des formulations de soin et de nettoyage.

**7.** Utilisation d'au moins un composé selon au moins l'une quelconque des revendications 1 à 3 ou d'au moins un polysiloxane pouvant être obtenu selon un procédé selon la revendication 4 comme agent de conditionnement pour des agents de traitement des cheveux et des agents de post-traitement des cheveux ainsi que comme agent pour améliorer la structure des cheveux.

**8.** Composition cosmétique, pharmaceutique ou dermatologique, en particulier formulations de soin et de nettoyage, agents de traitement des cheveux et agents de post-traitement des cheveux, aqueux, contenant un tensioactif, destinés à être éliminés par rinçage des cheveux ou à rester sur les cheveux, contenant au moins un composé selon au moins l'une quelconque des revendications 1 à 3 ou au moins un polysiloxane pouvant être obtenu selon un procédé selon la revendication 4.

**9.** Formulations de nettoyage et de soin pour le ménage, des utilisations industrielles et dans les collectivités, en particulier des adoucissants, contenant au moins un composé selon au moins l'une quelconque des revendications 1 à 3 ou au moins un polysiloxane pouvant être obtenu selon un procédé selon la revendication 4.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 1493384
- EP 0017122 A
- US 4895964 A
- DE 3719086
- EP 0294642 A
- EP 0530974 A
- EP 617607 A
- EP 1080714 A
- WO 2001082879 A
- US 6207141 B
- DE 3340708 A
- EP 1439200 A
- DE 102008041601
- DE 102007055485
- EP 1520870 A
- DE 102008001788
- DE 10327871


### In der Beschreibung aufgeführte Nicht-Patentliteratur


- **B. K. SCHRADER.** Grundlagen und Rezepturen der Kosmetika. Hüthig Buch Verlag Heidelberg, 329-341